# EUROPEAN PATENT APPLICATION

(11) **EP 2 309 272 A1**
(43) Date of publication of application: **13.04.2011**
(21) Application number: 09011876.1
(22) Date of filing: 17.09.2009
(51) Int. Cl.: G01N 33/68, C12N 15/10, G01N 33/50

(54) **Module-based vector useful in the assessment of antigen-specific T cell functions**

(71) Applicant: Charité Universitätsmedizin Berlin, 10117 Berlin (DE)
(72) Inventor: Stanke, Jonas, 14195 Berlin (DE); Hoffmann, Corinna, 13127 Berlin (DE); Kaufmann, Andreas M. Dr., 12200 Berlin (DE)
(74) Representative: Engelhard, Markus

(57) **Abstract**

The present invention refers to a module-based vector for concomitant eukaryotic expression of a proteasome degradable antigenic first protein and an accessory second protein, preferably a fluorescent reporter protein or a membrane-bound co-stimulatory protein. In particular, the module-based vector comprises a first nucleic acid sequence encoding the proteasome-degradable protein comprising a section encoding ubiquitin upstream of a section encoding the antigenic first protein, a second nucleic acid sequence encoding the accessory second protein, and a third nucleic acid sequence for encoupling the antigenic first and the accessory second protein which is located between the first and the second nucleic acid sequence. The present invention also refers to a use of the module-based vector for assessing antigen-specific T cell functions. The present invention further refers to a plasmid transfection fluorolysis (PFT) assay using the module-based vector.

SEQUENCE LISTING

<110>  Charité - Universitätsmedizin Berlin

<120>  Module-based vector useful in the assessment of antigen-specific
       T cell functions

<130>  FB22213

<160>  14

<170>  PatentIn version 3.3

<210>  1
<211>  8
<212>  PRT
<213>  Artificial

<220>
<223>  self-cleavage peptide sequence

<220>
<221>  VARIANT
<222>  (2)..(2)
<223>  Xaa can be any naturally occurring amino acid

<220>
<221>  VARIANT
<222>  (4)..(4)
<223>  Xaa can be any naturally occurring amino acid

<400>  1

Asp Xaa Glu Xaa Asn Pro Gly Pro
1               5


<210>  2
<211>  9
<212>  PRT
<213>  Artificial

<220>
<223>  CMV PP65 495-503

<400>  2

Asn Leu Val Pro Met Val Ala Thr Val
1               5


<210>  3
<211>  9
<212>  PRT
<213>  Artificial

<220>
<223>  HIV Pol 510-518

<400>  3

Ile Leu Lys Glu Pro Val His Gly Val
1               5


<210>  4
<211>  22
<212>  PRT
<213>  Artificial

<220>
<223>  self-cleavage peptide sequence foot and mouth disease virus

<400>  4

Pro Val Lys Gln Leu Leu Asn Phe Asp Leu Leu Lys Leu Ala Gly Asp
1               5                   10                  15


Val Glu Ser Asn Pro Gly
            20


<210>  5
<211>  19
<212>  PRT
<213>  Artificial

<220>
<223>  self-cleavage peptide sequence equine rhinitis A virus

<400>  5

Gln Cys Thr Asn Tyr Ala Leu Leu Lys Leu Ala Gly Asp Val Glu Ser
1               5                   10                  15


Asn Pro Gly



<210>  6
<211>  18
<212>  PRT
<213>  Artificial

<220>
<223>  self-cleavage peptide sequence porcine teschovirus-1

<400>  6

Ala Thr Asn Phe Ser Leu Leu Lys Gln Ala Gly Asp Val Glu Glu Asn
1               5                   10                  15


Pro Gly

<210>  7
<211>  17
<212>  PRT
<213>  Artificial

<220>
<223>  self-cleavage peptide sequence Thosea asigna virus

<400>  7

Glu Gly Arg Gly Ser Leu Leu Thr Cys Gly Asp Val Glu Glu Asn Pro
1               5                   10                  15

Gly

<210>  8
<211>  76
<212>  PRT
<213>  Artificial

<220>
<223>  Ubi

<400>  8

<210>  9
<211>  76
<212>  PRT
<213>  Artificial

<220>
<223>  Ubi.G>A76

<400> 9

Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Ala
65                  70                  75

<210> 10
<211> 18
<212> DNA
<213> Artificial

<220>
<223> MCS

<400> 10
gaattcttcg aacgatcg                                                    18

<210> 11
<211> 3114
<212> DNA
<213> Artificial

<220>
<223> pC-Ubi-MCS1-2A-MCS2

<400> 11
   tcaatattgg ccattagcca tattattcat tggttatata gcataaatca atattggcta      60
   ttggccattg catacgttgt atctatatca taatatgtac atttatattg gctcatgtcc     120
   aatatgaccg ccatgttggc attgattatt gactagttat taatagtaat caattacggg     180
   gtcattagtt catagcccat atatggagtt ccgcgttaca taacttacgg taaatggccc     240
   gcctggctga ccgcccaacg acccccgccc attgacgtca ataatgacgt atgttcccat     300
   agtaacgcca atagggactt tccattgacg tcaatgggtg gagtatttac ggtaaactgc     360
   ccacttggca gtacatcaag tgtatcatat gccaagtccg ccccctattg acgtcaatga     420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaataaccc 660
   cgccccgttg acgcaaatgg gcggtaggcg tgtacggtgg gaggtctata taagcagagg 720
   tcgtttagtg aaccgtcaga tcactagtag ctttattgcg gtagtttatc acagttaaat 780
   tgctaacgca gtcagtgctc gactgatcac aggtaagtat caaggttaca agacaggttt 840
   aaggaggcca atagaaactg ggcttgtcga gacagagaag attcttgcgt ttctgatagg 900
   cacctattgg tcttactgac atccactttg cctttctctc cacaggggta cccaccggcg 960
   tgcgcaagat ccaccatgca gatcttcgtg aagactctga ctggtaagac catcaccctc 1020
   gaggtggagc ccagtgacac catcgagaat gtcaaggcaa agatccaaga taaggaaggc 1080
   attcctcctg atcagcagag gttgatcttt gccggaaaac agctggaaga tggtcgtacc 1140
   ctgtctgact acaacatcca gaaagagtcc accttgcacc tggtactccg tctcagaggt 1200
   gccgaattct tcgaacgatc ggagggccgc ggcagcctgc tgacctgcgg cgacgtggag 1260
   gagaaccccg ggcccgctag cgagctcgat gagtttggac aaaccacaac tagaatgcag 1320
   tgaaaaaaat gctttatttg tgaaatttgt gatgctattg ctttatttgt aaccattata 1380
   agctgcaata aacaagttaa caacaacaat tgcattcatt ttatgtttca ggttcagggg 1440
   gaggtgtggg aggtttttta aagcaagtaa aacctctaca aatgtggtac ttaagagggg 1500
   gagaccaaag ggcgagacgt taaggcctca cgtgacatgt gagcaaaagg ccagcaaaag 1560
   gccaggaacc gtaaaaaggc cgcgttgctg gcgtttttcc ataggctccg cccccctgac 1620
   gagcatcaca aaaatcgacg ctcaagtcag aggtggcgaa acccgacagg actataaaga 1680
   taccaggcgt ttccccctgg aagctccctc gtgcgctctc ctgttccgac cctgccgctt 1740
   accggatacc tgtccgcctt tctcccttcg ggaagcgtgg cgctttctca tagctcacgc 1800
   tgtaggtatc tcagttcggt gtaggtcgtt cgctccaagc tgggctgtgt gcacgaaccc 1860
   cccgttcagc ccgaccgctg cgccttatcc ggtaactatc gtcttgagtc caacccggta 1920
   agacacgact tatcgccact ggcagcagcc actggtaaca ggattagcag agcgaggtat 1980
   gtaggcggtg ctacagagtt cttgaagtgg tggcctaact acggctacac tagaaggaca 2040
   gtatttggta tctgcgctct gctgaagcca gttaccttcg gaaaaagagt tggtagctct 2100
   tgatccggca aacaaaccac cgctggtagc gtggtttttt tgtttgcaag cagcagatta 2160
   cgcgcagaaa aaaaggatct caagaagatc ctttgatctt ttctacgggg tctgacgctc 2220
   agtggaacga aaactcacgt taagggattt tggtcatgcc gtctcagaag aactcgtcaa 2280
   gaaggcgata gaaggcgatg cgctgcgaat cgggagcggc gataccgtaa agcacgagga 2340
   agcggtcagc ccattcgccg ccaagctctt cagcaatatc acgggtagcc aacgctatgt 2400
   cctgatagcg gtccgccaca cccagccggc cacagtcgat gaatccagaa aagcggccat 2460
   tttccaccat gatattcggc aagcaggcat cgccatgggt cacgacgaga tcctcgccgt 2520
   cgggcatgct cgccttgagc ctggcgaaca gttcggctgg cgcgagcccc tgatgctctt 2580
   cgtccagatc atcctgatcg acaagaccgg cttccatccg agtacgtgct cgctcgatgc 2640
   gatgtttcgc ttggtggtcg aatgggcagg tagccggatc aagcgtatgc agccgccgca 2700
   ttgcatcagc catgatggat actttctcgg caggagcaag gtgagatgac aggagatcct 2760
   gccccggcac ttcgcccaat agcagccagt cccttcccgc ttcagtgaca acgtcgagca 2820
   cagctgcgca aggaacgccc gtcgtggcca gccacgatag ccgcgctgcc tcgtcttgca 2880
   gttcattcag ggcaccggac aggtcggtct tgacaaaaag aaccgggcgc ccctgcgctg 2940
   acagccggaa cacggcggca tcagagcagc cgattgtctg ttgtgcccag tcatagccga 3000
   atagcctctc cacccaagcg gccggagaac ctgcgtgcaa tccatcttgt tcaatcataa 3060
   tattattgaa gcatttatca gggttcgtct cgtcccggtc tcctcccatg catg 3114

<210> 12
<211> 3114
<212> DNA
<213> Artificial

<220>
<223> pC-Ubi-MCS1-2A-MCS2 complementary strand

<400> 12
catgcatggg aggagaccgg gacgagacga accctgataa atgcttcaat aatattatga 60

ttgaacaaga tggattgcac gcaggttctc cggccgcttg ggtggagagg ctattcggct 120

atgactgggc acaacagaca atcggctgct ctgatgccgc cgtgttccgg ctgtcagcgc 180

aggggcgccc ggttcttttt gtcaagaccg acctgtccgg tgccctgaat gaactgcaag 240

acgaggcagc gcggctatcg tggctggcca cgacgggcgt tccttgcgca gctgtgctcg 300

acgttgtcac tgaagcggga agggactggc tgctattggg cgaagtgccg gggcaggatc 360

tcctgtcatc tcaccttgct cctgccgaga aagtatccat catggctgat gcaatgcggc 420

ggctgcatac gcttgatccg gctacctgcc cattcgacca ccaagcgaaa catcgcatcg 480

agcgagcacg tactcggatg gaagccggtc ttgtcgatca ggatgatctg gacgaagagc 540
atcaggggct cgcgccagcc gaactgttcg ccaggctcaa ggcgagcatg cccgacggcg 600
aggatctcgt cgtgacccat ggcgatgcct gcttgccgaa tatcatggtg gaaaatggcc 660
gcttttctgg attcatcgac tgtggccggc tgggtgtggc ggaccgctat caggacatag 720
cgttggctac ccgtgatatt gctgaagagc ttggcggcga atgggctgac cgcttcctcg 780
tgctttacgg tatcgccgct cccgattcgc agcgcatcgc cttctatcgc cttcttgacg 840
agttcttctg agacggcatg accaaaatcc cttaacgtga gttttcgttc cactgagcgt 900
cagaccccgt agaaaagatc aaaggatctt cttgagatcc tttttttctg cgcgtaatct 960
gctgcttgca aacaaaaaaa ccaccgctac cagcggtggt ttgtttgccg gatcaagagct 1020
accaactctt tttccgaagg taactggctt cagcagagcg cagataccaa atactgtcct 1080
tctagtgtag ccgtagttag gccaccactt caagaactct gtagcaccgc ctacatacct 1140
cgctctgcta atcctgttac cagtggctgc tgccagtggc gataagtcgt gtcttaccgg 1200
gttggactca agacgatagt taccggataa ggcgcagcgg tcgggctgaa cggggggttc 1260
gtgcacacag cccagcttgg agcgaacgac ctacaccgaa ctgagatacc tacagcgtga 1320
gctatgagaa agcgccacgc ttcccgaagg gagaaaggcg gacaggtatc cggtaagcgg 1380
cagggtcgga acaggagagc gcacgaggga gcttccaggg ggaaacgcct ggtatcttta 1440
tagtcctgtc gggtttcgcc acctctgact tgagcgtcga tttttgtgat gctcgtcagg 1500
ggggcggagc ctatggaaaa acgccagcaa cgcggccttt ttacggttcc tggccttttg 1560
ctggcctttt gctcacatgt cacgtgaggc cttaacgtct cgccctttgg tctccccctc 1620
ttaagtacca catttgtaga ggttttactt gctttaaaaa acctcccaca cctccccctg 1680
aacctgaaac ataaaatgaa tgcaattgtt gttgttaact tgtttattgc agcttataat 1740
ggttacaaat aaagcaatag catcacaaat ttcacaaata aagcattttt ttcactgcat 1800
tctagttgtg gtttgtccaa actcatcgag ctcgctagcg ggcccggggt tctcctccac 1860
gtcgccgcag gtcagcaggc tgccgcggcc ctccgatcgt tcgaagaatt cggcacctct 1920
gagacggagt accaggtgca aggtggactc tttctggatg ttgtagtcag acagggtacg 1980
accatcttcc agctgttttc cggcaaagat caacctctgc tgatcaggag gaatgccttc 2040
cttatcttgg atctttgcct tgacattctc gatggtgtca ctgggctcca cctcgagggt 2100
gatggtctta ccagtcagag tcttcacgaa gatctgcatg gtggatcttg cgcacgccgg 2160
tgggtacccc tgtggagaga aaggcaaagt ggatgtcagt aagaccaata ggtgcctatc 2220

agaaacgcaa gaatcttctc tgtctcgaca agcccagttt ctattggcct ccttaaacct 2280

gtcttgtaac cttgatactt acctgtgatc agtcgagcac tgactgcgtt agcaatttaa 2340

ctgtgataaa ctaccgcaat aaagctacta gtgatctgac ggttcactaa acgacctctg 2400

cttatataga cctcccaccg tacacgccta ccgcccattt gcgtcaacgg ggcggggtta 2460

ttacgacatt ttggaaagtc ccgttgattt tggtgccaaa acaaactccc attgacgtca 2520

atggggtgga gacttggaaa tccccgtgag tcaaaccgct atccacgccc attggtgtac 2580

tgccaaaacc gcatcaccat ggtaatagcg atgactaata cgtagatgta ctgccaagta 2640

ggaaagtccc gtaaggtcat gtactgggca taatgccagg cgggccattt accgtcattg 2700

acgtcaatag ggggcggact tggcatatga tacacttgat gtactgccaa gtgggcagtt 2760

taccgtaaat actccaccca ttgacgtcaa tggaaagtcc ctattggcgt tactatggga 2820

acatacgtca ttattgacgt caatgggcgg gggtcgttgg gcggtcagcc aggcgggcca 2880

tttaccgtaa gttatgtaac gcggaactcc atatatgggc tatgaactaa tgaccccgta 2940

attgattact attaataact agtcaataat caatgccaac atggcggtca tattggacat 3000

gagccaatat aaatgtacat attatgatat agatacaacg tatgcaatgg ccaatagcca 3060

atattgattt atgctatata accaatgaat aatatggcta atggccaata ttga 3114

<210> 13
<211> 3103
<212> DNA
<213> Artificial

<220>
<223> pC-MCS2-2A-Ubi-MCS1

<400> 13
tcaatattgg ccattagcca tattattcat tggttatata gcataaatca atattggcta 60

ttggccattg catacgttgt atctatatca taatatgtac atttatattg gctcatgtcc 120

aatatgaccg ccatgttggc attgattatt gactagttat taatagtaat caattacggg 180

gtcattagtt catagcccat atatggagtt ccgcgttaca taacttacgg taaatggccc 240

gcctggctga ccgcccaacg acccccgccc attgacgtca ataatgacgt atgttcccat 300

agtaacgcca atagggactt tccattgacg tcaatgggtg gagtatttac ggtaaactgc 360

ccacttggca gtacatcaag tgtatcatat gccaagtccg ccccctattg acgtcaatga 420

cggtaaatgg cccgcctggc attatgccca gtacatgacc ttacgggact ttcctacttg 480

gcagtacatc tacgtattag tcatcgctat taccatggtg atgcggtttt ggcagtacac 540

caatgggcgt ggatagcggt ttgactcacg gggatttcca agtctccacc ccattgacgt 600

caatgggagt ttgttttggc accaaaatca acgggacttt ccaaaatgtc gtaataaccc 660

cgccccgttg acgcaaatgg gcggtaggcg tgtacggtgg gaggtctata taagcagagg 720

tcgtttagtg aaccgtcaga tcactagtag ctttattgcg gtagtttatc acagttaaat 780

tgctaacgca gtcagtgctc gactgatcac aggtaagtat caaggttaca agacaggttt 840

aaggaggcca atagaaactg ggcttgtcga gacagagaag attcttgcgt ttctgatagg 900

cacctattgg tcttactgac atccactttg cctttctctc cacaggggta cccaccggcg 960

tgctagcgag ctcgagggcc gcggcagcct gctgacctgc ggcgacgtgg aggagaaccc 1020

cgggcccatg cagatcttcg tgaagactct gactggtaag accatcaccc tcgaggtgga 1080

gcccagtgac accatcgaga atgtcaaggc aaagatccaa gataaggaag gcattcctcc 1140

tgatcagcag aggttgatct ttgccggaaa acagctggaa gatggtcgta ccctgtctga 1200

ctacaacatc cagaaagagt ccaccttgca cctggtactc cgtctcagag gtgccgaatt 1260

cttcgaacga tcgtgagatg agtttggaca aaccacaact agaatgcagt gaaaaaaatg 1320

ctttatttgt gaaatttgtg atgctattgc tttatttgta accattataa gctgcaataa 1380

acaagttaac aacaacaatt gcattcattt tatgtttcag gttcaggggg aggtgtggga 1440

ggttttttaa agcaagtaaa acctctacaa atgtggtact taagaggggg agaccaaagg 1500

gcgagacgtt aaggcctcac gtgacatgtg agcaaaaggc cagcaaaagg ccaggaaccg 1560

taaaaaggcc gcgttgctgg cgtttttcca taggctccgc ccccctgacg agcatcacaa 1620

aaatcgacgc tcaagtcaga ggtggcgaaa cccgacagga ctataaagat accaggcgtt 1680

tccccctgga agctccctcg tgcgctctcc tgttccgacc ctgccgctta ccggatacct 1740

gtccgccttt ctcccttcgg gaagcgtggc gctttctcat agctcacgct gtaggtatct 1800

cagttcggtg taggtcgttc gctccaagct gggctgtgtg cacgaacccc ccgttcagcc 1860

cgaccgctgc gccttatccg gtaactatcg tcttgagtcc aacccggtaa gacacgactt 1920

atcgccactg gcagcagcca ctggtaacag gattagcaga gcgaggtatg taggcggtgc 1980

tacagagttc ttgaagtggt ggcctaacta cggctacact agaaggacag tatttggtat 2040

ctgcgctctg ctgaagccag ttaccttcgg aaaaagagtt ggtagctctt gatccggcaa 2100

acaaaccacg ctggtagcgg tggttttttt gtttgcaagc agcagattac gcgcagaaaa 2160

aaaggatctc aagaagatcc tttgatcttt tctacggggt ctgacgctca gtggaacgaa 2220

aactcacgtt aagggatttt ggtcatgccg tctcagaaga actcgtcaag aaggcgatag 2280

aaggcgatgc gctgcgaatc gggagcggcg ataccgtaaa gcacgaggaa gcggtcagcc 2340

cattcgccgc caagctcttc agcaatatca cgggtagcca acgctatgtc ctgatagcgg 2400

tccgccacac ccagccggcc acagtcgatg aatccagaaa agcggccatt ttccaccatg 2460

atattcggca agcaggcatc gccatgggtc acgacgagat cctcgccgtc gggcatgctc 2520

gccttgagcc tggcgaacag ttcggctggc gcgagcccct gatgctcttc gtccagatca 2580

tcctgatcga caagaccggc ttccatccga gtacgtgctc gctcgatgcg atgtttcgct 2640

tggtggtcga atgggcaggt agccggatca agcgtatgca gccgccgcat tgcatcagcc 2700

atgatggata ctttctcggc aggagcaagg tgagatgaca ggagatcctg ccccggcact 2760

tcgcccaata gcagccagtc ccttcccgct tcagtgacaa cgtcgagcac agctgcgcaa 2820

ggaacgcccg tcgtggccag ccacgatagc cgcgctgcct cgtcttgcag ttcattcagg 2880

gcaccggaca ggtcggtctt gacaaaaaga accgggcgcc cctgcgctga cagccggaac 2940

acggcggcat cagagcagcc gattgtctgt tgtgcccagt catagccgaa tagcctctcc 3000

acccaagcgg ccggagaacc tgcgtgcaat ccatcttgtt caatcataat attattgaag 3060

catttatcag ggttcgtctc gtcccggtct cctcccatgc atg 3103

<210> 14
<211> 3103
<212> DNA
<213> Artificial

<220>
<223> pC-MCS2-2A-Ubi-MCS1 complementary strand

<400> 14
catgcatggg aggagaccgg gacgagacga accctgataa atgcttcaat aatattatga 60

ttgaacaaga tggattgcac gcaggttctc cggccgcttg ggtggagagg ctattcggct 120

atgactgggc acaacagaca atcggctgct ctgatgccgc cgtgttccgg ctgtcagcgc 180

aggggcgccc ggttcttttt gtcaagaccg acctgtccgg tgccctgaat gaactgcaag 240

acgaggcagc gcggctatcg tggctggcca cgacgggcgt tccttgcgca gctgtgctcg 300

acgttgtcac tgaagcggga agggactggc tgctattggg cgaagtgccg gggcaggatc 360

tcctgtcatc tcaccttgct cctgccgaga aagtatccat catggctgat gcaatgcggc 420

ggctgcatac gcttgatccg gctacctgcc cattcgacca ccaagcgaaa catcgcatcg 480

agcgagcacg tactcggatg gaagccggtc ttgtcgatca ggatgatctg gacgaagagc 540

atcaggggct cgcgccagcc gaactgttcg ccaggctcaa ggcgagcatg cccgacggcg 600

aggatctcgt cgtgacccat ggcgatgcct gcttgccgaa tatcatggtg gaaaatggcc 660

gcttttctgg attcatcgac tgtggccggc tgggtgtggc ggaccgctat caggacatag 720

cgttggctac ccgtgatatt gctgaagagc ttggcggcga atgggctgac cgcttcctcg 780

tgctttacgg tatcgccgct cccgattcgc agcgcatcgc cttctatcgc cttcttgacg 840

agttcttctg agacggcatg accaaaatcc cttaacgtga gttttcgttc cactgagcgt 900

cagaccccgt agaaaagatc aaaggatctt cttgagatcc tttttttctg cgcgtaatct 960

gctgcttgca aacaaaaaaa ccaccgctac cagcggtggt ttgtttgccg gatcaagagct 1020

accaactctt tttccgaagg taactggctt cagcagagcg cagataccaa atactgtcct 1080

tctagtgtag ccgtagttag gccaccactt caagaactct gtagcaccgc ctacatacct 1140

cgctctgcta atcctgttac cagtggctgc tgccagtggc gataagtcgt gtcttaccgg 1200

gttggactca agacgatagt taccggataa ggcgcagcgg tcgggctgaa cggggggttc 1260

gtgcacacag cccagcttgg agcgaacgac ctacaccgaa ctgagatacc tacagcgtga 1320

gctatgagaa agcgccacgc ttcccgaagg gagaaaggcg gacaggtatc cggtaagcgg 1380

cagggtcgga acaggagagc gcacgaggga gcttccaggg ggaaacgcct ggtatcttta 1440

tagtcctgtc gggtttcgcc acctctgact tgagcgtcga tttttgtgat gctcgtcagg 1500

ggggcggagc ctatggaaaa acgccagcaa cgcggccttt ttacggttcc tggccttttg 1560

ctggcctttt gctcacatgt cacgtgaggc cttaacgtct cgccctttgg tctccccctc 1620

ttaagtacca catttgtaga ggttttactt gctttaaaaa acctcccaca cctccccctg 1680

aacctgaaac ataaaatgaa tgcaattgtt gttgttaact tgtttattgc agcttataat 1740

ggttacaaat aaagcaatag catcacaaat ttcacaaata aagcattttt ttcactgcat 1800

tctagttgtg gtttgtccaa actcatctca cgatcgttcg aagaattcgg cacctctgag 1860

acggagtacc aggtgcaagg tggactcttt ctggatgttg tagtcagaca gggtacgacc 1920

atcttccagc tgttttccgg caaagatcaa cctctgctga tcaggaggaa tgccttcctt 1980

atcttggatc tttgccttga cattctcgat ggtgtcactg ggctccacct cgagggtgat 2040

ggtcttacca gtcagagtct tcacgaagat ctgcatgggc ccggggttct cctccacgtc 2100

gccgcaggtc agcaggctgc cgcggccctc gagctcgcta gcacgccggt gggtacccct 2160

gtggagagaa aggcaaagtg gatgtcagta agaccaatag gtgcctatca gaaacgcaag 2220

aatcttctct gtctcgacaa gcccagtttc tattggcctc cttaaacctg tcttgtaacc 2280

ttgatactta cctgtgatca gtcgagcact gactgcgtta gcaatttaac tgtgataaac 2340

taccgcaata aagc[?]actag tgatctgacg gttcactaaa cgacctctgc ttatatagac 2400

ctcccaccgt acacgcctac cgcccatttg cgtcaacggg gcggggttat tacgacattt 2460

tggaaagtcc cgttgatttt ggtgccaaaa caaactccca ttgacgtcaa tggggtggag 2520

acttggaaat ccccgtgagt caaaccgcta tccacgccca ttggtgtact gccaaaaccg 2580

catcaccatg gtaatagcga tgactaatac gtagatgtac tgccaagtag gaaagtcccg 2640

taaggtcatg tactgggcat aatgccaggc gggccattta ccgtcattga cgtcaatagg 2700

gggcggactt ggcatatgat acacttgatg tactgccaag tgggcagttt accgtaaata 2760

ctccacccat tgacgtcaat ggaaagtccc tattggcgtt actatgggaa catacgtcat 2820

tattgacgtc aatgggcggg ggtcgttggg cggtcagcca ggcgggccat ttaccgtaag 2880

ttatgtaacg cggaactcca tatatgggct atgaactaat gaccccgtaa ttgattacta 2940

ttaataacta gtcaataatc aatgccaaca tggcggtcat attggacatg agccaatata 3000

aatgtacata ttatgatata gatacaacgt atgcaatggc caatagccaa tattgattta 3060

tgctatataa ccaatgaata atatggctaa tggccaatat tga 3103

## Description

The present invention refers to a module-based vector for concomitant eukaryotic expression of a proteasome degradable antigenic protein and an independent protein, preferably a fluorescent reporter protein or a membrane-bound co-stimulatory protein. The present invention further refers to a flow-cytometry-based plasmid transfection fluorolysis (PFT) assay for the detection of activated T cells.

### Background of the invention

The adaptive immune system depends on cytotoxic CD8+ T cells (CTL) and CD4+ helper and regulatory T cells for effective immune responses and recall. Antigen-specific effects require specific interactions mediated by the T cell receptor (TCR) from the T cell side, and an immunogenic peptide presented by a major histocompatibility complex (MHC)/human leukocyte antigen (HLA) as well as co-stimulatory molecules from the side of an antigen-presenting cell (APC). To functionally describe low frequencies of effector T cells routinely in-vitro, e.g. tumor-specific T cells (1) in the peripheral blood or so called tumor infiltrating lymphocytes (TIL), methods have to be highly sensitive (2). They might include initial steps to enrich antigen-specific T cells while maintaining clonal diversity of antigen-specific T cell populations. These principles should allow to newly define immunogenic epitopes of a given antigenic protein and/or to monitor e.g. vaccination success or auto-immunity.

Functional description of effector T cells often requires large cell numbers and/or relies on surrogate parameters assessing the potential of a given cell without direct measurement of specific functions. Typically, low frequencies of antigen-specific cells accompany small samples with overall low cell numbers and they must be expanded to meet the detection limits of established methods. Substances like phytohemagglutinine, phorbol esters or high doses of CD3-specific antibody non-specifically crosslink T cells efficiently activating these cells and inducing T cell proliferation in-vitro. In cultures like these, specificities for given antigens as measured by antigen-specific effector functions are lost upon re-stimulation (3). As in-vivo, in-vitro recovery of functional T cells requires antigen-specific stimuli provided by MHC/HLA-restricted immunogenic peptides and co-stimulatory molecules (4). Absence of co-stimulators molecules e.g. of B7-1 (CD80) and/or B7-2 (CD86) during interactions of a HLA-presented peptide and the respective TCR is known to cause T cell anergy (5,6).

There are only a few types of potential professional APC, e.g. B cells, macrophages or dendritic cells (DC). Although easily obtained e.g. from the peripheral blood, overall frequencies of APC and their precursors are typically below 10% of all peripheral blood mononuclear cells (PBMC). Generation of sufficient amounts of APC and of autologous APC in particular can be very time consuming and expensive. Transformation of B cells with the Epstein Barr virus (EBV) produces immortalized B lymphoblastic cell lines (B-LCL) useful in antigen-specific T-cell activation and expansion if the antigen of interest was at least as strongly immunogenic as the EBV antigens produced by the transforming virus (7). In addition, it often takes about 4-6 weeks to obtain B-LCL from autologous PBMC. Therefore different efforts have been made to generate 'universal' APC artificially by altering partially HLA-matched permanent cell lines to constitutively express co-stimulatory molecules, e.g. CD80 or CD86 for antigen-specific T-cell stimulation (8,9). Immunogenicity of tumor cells can be enhanced by ectopic expression of CD80 or CD86 molecules (10-12).

Induction and examination of antigen-specific CTL using exogenous synthetic HLA-binding peptide epitopes have many disadvantages. Candidate epitopes have to have a high affinity to a previously known HLA subtype to replace peptides from the preformed HLA/peptide complexes on the APC surface. This affinity can be predicted theoretically by the use of databases like BIMAS or SYFPEITHI assembling binding properties of MHC/HLA molecules (13). A major disadvantage of the approach to load external peptides is that even peptides with low affinity to given MHC/HLA molecules might induce strong T cell reactions but are not efficiently presented (14). In contrast, by endogenous expression, antigenic peptides are continuously produced and loaded on to MHC molecules. Loading prolonged peptides to be trimmed by the APC after internalization is expansive and inefficient especially in cells with limited capabilities for endocytosis (15). Insufficient presentation of e.g. minor antigenic structures during in-vitro restimulation to increase the number of effector T cells results in frequency shifts that alter the composition of the expanded T cell population compared to the initial situation with respect to antigen-specific T cells (3).

On the other hand, established assays for functional description of effector T cells can be limited by the demand for large numbers of antigen-specific T cells. Therefore these methods are often restricted to strong T cell-mediated immune answers and/or assess surrogate parameters showing rather the potential of a given cell population than the effector functions themselves.

The enzyme-linked immunosorbent spot (ELISpot) assay is a widely distributed method to assess antigen specific CTL activation but it only describes surrogate parameters like inflammatory cytokine release. During the procedure, cytokines are captured in the immediate vicinity of producing cells if cells are cultured on a membrane coated with an antibody specific for a defined cytokine. In the second step, membrane-bound cytokines are detected using a secondary antibody labeled e.g. for chromogenic substrate conversion (16,17). Hence the name of the assay, each coloured spot represents one cytokine-producing cell and the spot number in correlation to the initial cell number quantifies potential effector cells within the cell population to be assessed. Unfortunately, interferon (IFN)γ, the most common cytokine to be routinely assessed for pro-inflammatory cells is produced by various subsets of immune cells (18). Thus, the ELISpot assay does not discriminate IFNγ-producing effector T cells e.g. from macrophages if mixed cell populations are questioned. Although this side effect can be minimized by prior enrichment of antigen-specific T cells, the ELISpot assay describes T cell function only indirectly and the actual effect of CTL, the antigen-specific target cell lysis, is not covered.

Similar to the ELISpot assay, flow cytometric assessment of intracellular cytokines (IC-FACS) measures the potential of single cells. Upon a specific stimulus, cells produce pro-inflammatory cytokines like IFNγ or tumor necrosis factor (TNF) α. Since secretion is blocked e.g. by brefeldin A, newly expressed cytokines remain within the producing cell. After permeabilization of the cell membranes, intracellular cytokines can be stained with fluorescently labeled specific antibodies. The IC-FACS can be combined e.g. with T cell surface makers including MHC/HLA tetramers consisting of recombinant peptide loaded MHC/HLA complexes, tetramerized via fluorescently labeled (strept)avidine to directly stain antigen-specific TCR (16,19). Although this combination allows describing the subtype of individual cytokine-producing cells, this method again only assess the frequency of the antigen-specific T cells, but not their functionality.

The classic chromium release assay (CRA) directly measures the lysis of target cells radioactively labeled with [⁵¹Cr] by uptake of [⁵¹Cr]-sodium dichromate by CTL or natural killer (NK) cells (16,20). The extent of effector cell-mediated lysis correlates to the release of target cell-bound radioactivity into the cell culture medium after the incubation of labeled target cells with the T cell populations for 4 h or 18 h to measure essentially CTL or NK cell activity. The CRA is considered the 'gold standard' to directly describe CTL function diagnostically or for scientific research questions. Use of radioactive material and the time-consuming need to expand antigen-specific CTL populations of large amounts of antigen-specific effector CTL make poor standardization characteristics and limit the CRA in routine clinical use especially for antigens with low immunogenicity. The lactate dehydrogenase (LDH) assay is the non-radioactive equivalent of the CRA and also determines only the functionality of CTL. The LDH, an ubiquitous enzyme from the glycolysis catalysing the oxidation of lactate to pyruvate and located in the cytoplasm of viable cells is released from the target cells as a result of the CTL activity. The amount of target cell-released LDH corresponds to the conversion of a chromogenic substrate used for readout (21). Since all cells contain LDH this experimental approach demands even larger amounts of CTL than the CRA to exclude spontaneous LDH release from destructed effector T cells making this assay not only less sensitive than the CRA but requiring even more effort to obtain sufficient amounts of antigen-specific CTL.

In the VITAL assay, cells are first loaded with fluorescent labels and then incubated with exogenous synthetic peptides to obtain target cells. Antigen-specific target cells receive a peptide that is immunogenic in a given MHC/HLA context. Cells labeled with a different fluorogenic dye are treated with a peptide known to be non-immunogenic against the same MHC/HLA background as the specific peptide above to produce control target cells. Specific and control target cells are co-cultured with cell populations to be questioned for antigen-specific CTL. After incubation antigen-specific target cell lysis can be determined by the ratio of the remaining control and target cells, which are quantified by flow cytometry (22-26). Although in general further markers accessible to flow cytometric analysis might complement direct measurement of CTL function, major disadvantages of the VITAL assay arise from toxic side effects of fluorogenic dyes and in particular from the necessity for previous detailed knowledge of MHC/HLA molecules and of the respective immunogenic peptide epitopes of a given antigenic protein.

The object of the present invention is to provide a sensitive method for the detection of activated T cells as well as means useful in this method.

### Summary of the invention

The object of the present invention is solved by a module-based vector for concomitant expression of an antigenic first protein and an accessory second protein, the vector comprising:
(a) a first nucleic acid sequence encoding a proteasome-degradable protein comprising a section encoding ubiquitin upstream of a section encoding the antigenic first protein;
(b) a second nucleic acid sequence encoding the accessory second protein;
(c) a third nucleic acid sequence for encoupling the antigenic first and the accessory second protein;
   wherein the third nucleic acid sequence is located between the first and the second nucleic acid sequence; and
(d) a first promoter for eukaryotic expression which is located upstream of the first, second and third nucleic acid sequence.

The object of the present invention is further solved by a module-based vector for concomitant expression of an antigenic first protein and an accessory second protein, the vector comprising:
(a) a first nucleic acid sequence encoding a proteasome-degradable protein comprising a section encoding ubiquitin upstream of a section encoding the antigenic first protein;
(b) a second nucleic acid sequence encoding the accessory second protein which is a reporter protein, preferably a fluorescent reporter protein;
(c) a third nucleic acid sequence for encoupling the antigenic first and the accessory second protein;
   wherein the third nucleic acid sequence is located between the first and the second nucleic acid sequence; and
(d) a first promoter for eukaryotic expression which is located upstream of the first, second and third nucleic acid sequence.

The object of the present invention is further solved by a module-based vector for concomitant expression of an antigenic first protein and an accessory second protein, the vector comprising:
(a) a first nucleic acid sequence encoding a proteasome-degradable protein comprising a section encoding ubiquitin upstream of a section encoding the antigenic first protein;
(b) a second nucleic acid sequence encoding the accessory second protein which is membrane-bound co-stimulatory protein;
(c) a third nucleic acid sequence for encoupling the antigenic first and the accessory second protein;
   wherein the third nucleic acid sequence is located between the first and the second nucleic acid sequence; and
(d) a first promoter for eukaryotic expression which is located upstream of the first, second and third nucleic acid sequence.

In one embodiment, the first nucleic acid sequence is located upstream of the third nucleic acid sequence and the second nucleic acid sequence is located downstream of the third nucleic acid sequence.

In one embodiment, the first nucleic acid sequence is located downstream of the third nucleic acid sequence and the second nucleic acid sequence is located upstream of the third nucleic acid sequence.

In one embodiment, the third nucleic acid sequence is encoding an amino acid sequence comprising a self-cleavage peptide sequence.

In one embodiment, the self-cleavage peptide sequence has an amino acid sequence according to SEQ ID NO: 1, i.e. DxExNPGP (Asp Xaa Glu Xaa Asn Pro Gly Pro), wherein x (Xaa) can be any naturally occurring amino acid. The cleavage occurs between the glycine and proline residues.

In one embodiment, the self-cleavage peptide sequence is a Picornavirus 2A self-cleavage sequence.

In one embodiment, the third nucleic acid is encoding an mRNA comprising an internal ribosomal entry site (IRES).

In one embodiment, the antigenic first protein is selected from the group comprising a tumor associated, viral, bacterial and autoantigenic protein. The term "tumor associated" means that the protein is expressed and optionally presented, exposed and/or secreted by tumor cells.

In one embodiment, the antigenic first protein is a full-length protein, preferably selected from the group comprising CMVpp65, IMP M1, and HPV16E7.

In one embodiment, the ubiquitin consists of a native ubiquitin amino acid sequence or comprises a mutated ubiquitin amino acid sequence, preferably a mutated ubiquitin amino acid sequence with the C-terminal glycine residue being exchanged for an alanine residue.

In one embodiment, the first nucleic acid sequence further comprises a section encoding a tag protein, preferably selected from the group comprising a hemagglutinine-tag, myc-tag and 6xHis-tag. Preferably, the section encoding the tag protein is located downstream of the section encoding the antigenic protein.

In one embodiment, the promoter is selected from the group comprising a FerL, FerH, EF1, CMV, HTLV, Ubi, RSV, β-actin, and SV40 promoter.

In one embodiment, the vector further comprises a viral second promoter, preferably a T7 promoter, which is located upstream of the first, second and third nucleic acid sequence. Preferably, the viral second promoter is located downstream of the first promoter.

In one embodiment of the vector, which is a module-based reporter vector (RV), the accessory second protein is a reporter protein, preferably a fluorescent reporter protein, most preferably selected from the group comprising GFP, dsRed, Yellow and a recombinant combination thereof.

In one embodiment, the reporter protein is GFP and the antigenic first protein is CMVpp65 or HPV16E7. In this embodiment, the self-cleavage peptide sequence preferably is a Picomavirus 2A self-cleavage peptide sequence.

In one embodiment of the vector, which is a module-based stimulation vector (SV), the accessory second protein is a membrane-bound co-stimulatory protein for antigen-specific T cell activation, preferably selected from the group comprising CD80, CD86, GITR-L, CD54, CD40, OX40 and 4-1BB.

In one embodiment, the membrane-bound co-stimulatory protein is CD86 and the antigenic first protein is CMVpp65 or HPV16E6. In this embodiment, the self-cleavage peptide sequence preferably is a Picornavirus 2A self-cleavage peptide sequence.

In one embodiment, the first nucleic acid sequence encoding CMVpp65 is located upstream of the third nucleic acid sequence, and the second nucleic acid sequence encoding CD86 is located downstream of the third nucleic acid sequence.

The object of the present invention is further solved by a target cell for cytotoxic CD8⁺ cells (CTL) comprising a module-based vector (RV) according to the present invention.

The object of the present invention is further solved by a pair of target cells according to the present invention consisting of a first target cell which is a test target cell, and a second target cell which is a control target cell, wherein the control target cell differs from the test target cell in the antigenic first protein and in the reporter protein.

In one embodiment of the pair of target cells, the reporter protein of the test target cell is GFP and the reporter protein of the control target cell is dsRed.

The object of the present invention is further solved by an antigen-presenting cell comprising a module-based vector (SV) according to the present invention.

The object of the present invention is further solved by a method for preparing a target cell according to the present invention comprising the step of transfecting a cell with a module-based vector (RV) according to the present invention.

The object of the present invention is further solved by a method for preparing an antigen-presenting cell according to the present invention comprising the step of transfecting a cell with a module-based vector (SV) according to the present invention.

In one embodiment of the method for preparing a target cell or an antigen presenting cell the step of transfecting the cell comprises a chemical precipitation, lipofection, electroporation or nucleofection, preferably a nucleofection.

In one embodiment of the method for preparing a target cell or an antigen presenting cell the cell to be transfected is selected from the group comprising B-LCL, K562, K562-A2, EL-4, and peripheral blood mononuclear cells.

The object of the present invention is further solved by a use of a module-based vector (RV) according to the present invention, or of a target cell according to the present invention for studying T cell functions ex-vivo, preferably for detecting and monitoring T cells activated by a vaccine, an infectious agent, preferably a virus, an allergen or an autoantigen.

The object of the present invention is further solved by a use of a module-based vector (SV) according to the present invention, or an antigen-presenting cell according to the present invention for activating and/or expanding T cells.

The object of the present invention is further solved by a plasmid transfection fluorolysis (PTF) assay comprising the following steps:
(a) providing one, two or more population(s) of a test target cell according to the present invention, preferably by using a method according to the present invention;
(b) providing a population of a control target cell according to the present invention, preferably by using a method according to the present invention;
(c) providing a population of preferably pre-activated CTL;
(d) mixing the populations of (a), (b) and (c);
(e) co-culturing the populations of (a), (b) and (c);
(f) measuring by fluorescence spectroscopy, preferably by FACS, the extent of fluorescence for the one, two or more population(s) of a test target cell and the extent of fluorescence for the population of a control target cell;
(g) determining the ratio of the extent of fluorescence measured for each of the one, two or more population(s) of a test target cell and the extent of fluorescence measured for the population of a control target cell.

In one embodiment, the CTL are pre-activated by an antigenic protein expressed by the one, two or more population(s) of an antigen-presenting cell according to the present invention.

In one embodiment, step (e) or step (e') below, i.e. the step of co-culturing, is carried out for up to 96 h, preferably for 72 h. Alternatively, co-culturing can be carried out for up to one week.

In one embodiment, the assay further comprises the following steps:
(a') providing the one, two or more population(s) of a test target cell as in step (a) above;
(b') providing the population of a control target cell as in step (b) above;
(d') mixing the populations of (a') and (b');
(e') co-culturing the populations of (a') and (b');
(f') measuring by fluorescence spectroscopy, preferably by FACS, the extent of fluorescence for the one, two or more population(s) of a test target cell and the extent of fluorescence for the population of a control target cell;
(g') determining the ratio of the extent of fluorescence measured for each of the one, two or more population(s) of a test target cell and the extent of fluorescence measured for the population of a control target cell;
(i) calculating an antigen-specific target cell lysis from the ratio determined in step (g) above and the ratio determined in step (g').

In one embodiment of the assay the two or more populations of a test target cell differ from each other in the antigenic first protein and in the reporter protein.

In one embodiment of the assay the CTL are pre-activated with an antigen-presenting cell according to the present invention, wherein the antigenic protein expressed by the antigen-presenting cell is the same as the antigenic protein expressed by the test target cell.

The object of the present invention is further solved by a test kit comprising a module-based vector according to the present invention and/or a target cell according to the present invention and/or a pair of target cells according to the present invention and/or an antigen-presenting cell according to the present invention and, optionally, a sheet providing instructions for carrying out a method according to the present invention.

The object of the present invention is further solved by a module-based vector comprising a nucleic acid sequence according to SEQ ID NO: 11 and/or SEQ ID NO: 12.

The object of the present invention is further solved by a module-based vector comprising a nucleic acid sequence according to SEQ ID NO: 13 and/or SEQ ID NO: 14.

The wording "a nucleic acid sequence for encoupling a first and a second protein" means that the nucleic acid is capable of providing for a cleavage and separation of the two proteins concomitantly expressed.

The term "a test target cell" refers to a cell expressing an antigenic protein of interest.

The term "a control target cell" refers to a cell expressing an irrelevant antigenic protein.

We herein describe module-based stimulation (SV) and reporter vectors (RV) for concomitant expression of a proteasome-degradable protein (PDP) with upstream ubiquitin (Ubi) and a functionally independent protein that might be a membrane-bound co-stimulatory or a fluorescent reporter (C/R) protein to activate and expand antigen-specific T cells and/or to assess T cell effector functions. Both protein entities are functionally uncoupled via a fusion protein that contains a self-cleavage peptide sequence or via a fused mRNA with an internal ribosomal entry site (IRES). As for antigen-specific in-vitro activation and expansion of T cells, APC used in co-cultures are transfected by a SV comprising coding sequences for a specific antigenic PDP and a functionally uncoupled membrane-bound co-stimulatory protein. As for description of CTL activity, potential target cells are transfected by RV coding for a fluorescent reporter protein functionally uncoupled specific antigenic PDP or a control PDP. A flow cytometry-based plasmid transfection fluorolysis (PTF) assays set up with at least one type of antigen-specific target cells in conjunction with at least one type of control target cells measures antigen-specific target cell lysis from the relation of antigen-specific to control target cells.

The modular vectors disclosed herein provide the technical background for a direct, highly sensitive, non-radioactive and versatile method e.g. to diagnostically detect and monitor functional T cells induced by vaccination or viral infections e.g. by CMV, Hepatitis B or C virus (HBV, HCV), human papilloma virus (HPV), EBV and human immunodeficiency virus (HIV), by allergens or by autoantigens e.g. mediating multiple sclerosis, ankylosing spondy-lites and diabetes via their effector function. This approach uses natural mechanisms for presentation of a processed antigen of interest. As for basic research issues the strategy using the disclosed vectors allows e.g. to define new antigenic proteins, to directly assess the antigenicity of proteasome-processed proteins, and to define minimum T cell epitopes and their dependency on MHC/HLA subsets.

### Detailed description of the invention

- Figure 1:: Design of the modular vector system using a 2A self-cleavage site to functionally uncouple recombinant proteins for antigen-specific T cell description. P, pro- moter; Ubi, ubiquitin; (tag), peptide tag (optional); 2A, protein self-cleavage site; C/R, membrane-bound co-stimulatory or fluorescent reporter protein.
- Figure 2:: Minimum prerequisite peptide sequences for 2A self-cleavage sites with exam- ples. (1) Foot and mouth disease virus (SEQ ID NO: 4). (2) Equine rhinitis A vi- rus (SEQ ID NO: 5). (3) Porcine teschovirus-1 (SEQ ID NO: 6). (4) Thosea asigna virus (SEQ ID NO: 7). (M) minimum requisite amino acid residues for self-cleavage.
- Figure 3:: Design of the modular vector system using an IRES sequence to functionally un- couple recombinant proteins for antigen-specific T cell description. P, promoter; Ubi, ubiquitin; (tag), peptide tag (optional); IRES, internal ribosomal entry site; C/R, membrane-bound co-stimulatory or fluorescent reporter protein.
- Figure 4:: Minimum prerequisite Ubi protein and/or nucleotide sequences with examples. Ubi (SEQ ID NO: 8); Ubi.G>A76 (SEQ ID NO: 9).
- Figure 5:: Schematic vector background and MCS sequence (SEQ ID NO: 10). The expres- sion cassette is according to Figs. 1 and 3.
- Figure 6:: Effect of Ubi on proteasomal degradation of downstream fused GFP. K562 trans- fected for expression.
- Figure 7:: Comparison of CD8+ T cells stimulated with APC loaded with MHC-restricted exogenous synthetic CMVpp65₄₉₅₋₅₀₃ or transfected with a recombinant ubiquiti- nated CMVpp65. DCs as APC in co-culture.
- Figure 8:: Effect of IRES and the 2A self-cleavage site on degradation of downstream fused GFP. K562 transfected for expression.
- Figure 9:: Comparison of CD8+ T cells stimulated with APC transfected with a recombinant ubiquitinated CMVpp65 in combination with membrane-bound co-stimulatory CD86. Stimulating APC transfected for expression.
- Figure 10:: Principle and exemplary set-up of standard PTF assays.
- Figure 11:: Principle and exemplary set-up of duplex PTF assays.
- Figure 12:: Principle of multiplex PTF assays.
- Figure 13:: Comparison of the sensitivity of standard PTF assay and CRA to detect CTL spe- cific for CMVpp65.
- Figure 14:: Comparison of the sensitivity of standard PTF and VITAL assay to detect CMVpp65-specific CTL.
- Figure 15:: Principle and schedule for low frequency effector T cells comprising antigen- specific effector T cell enrichment and subsequent PTF assay.
- Figure 16:: PTF assay for detection of HPV16E7-specific CTL from a model of C57/BL6 mice immunized using a recombinant Adenovirus vaccine carrying HPV 16E7.
- Figure 17:: PTF assay for detection of HPV16E6-specific TIL from a HPV tumor patient.

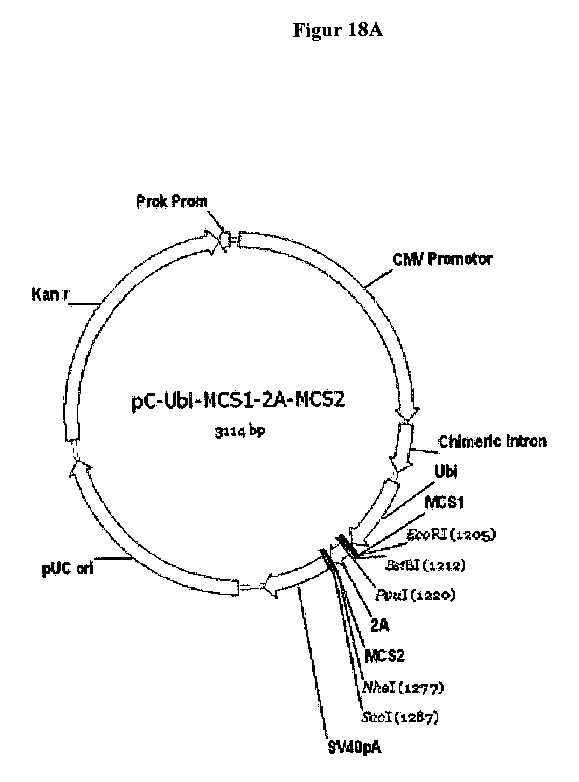

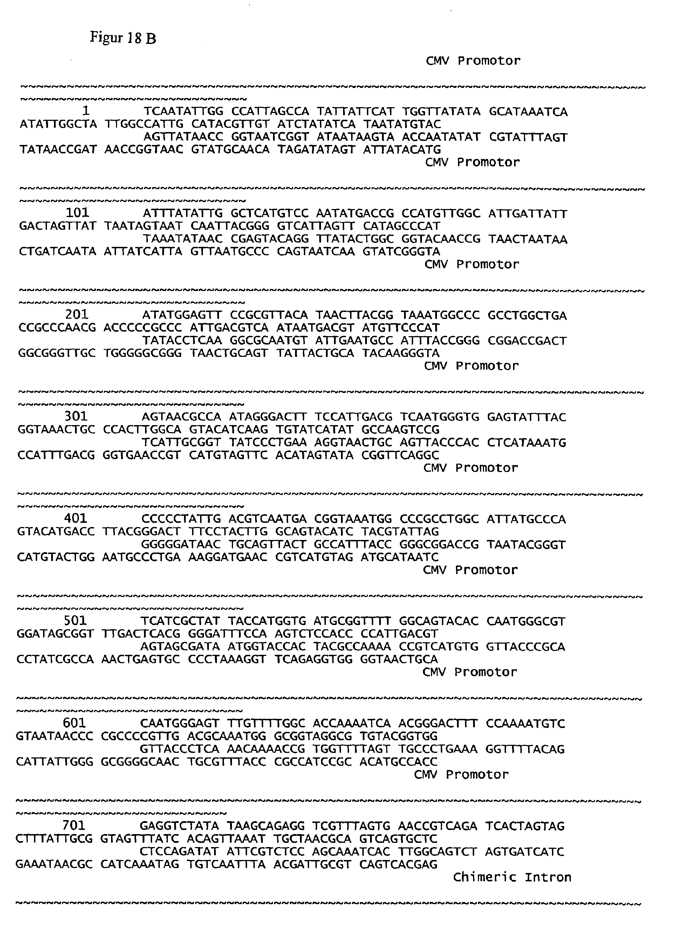

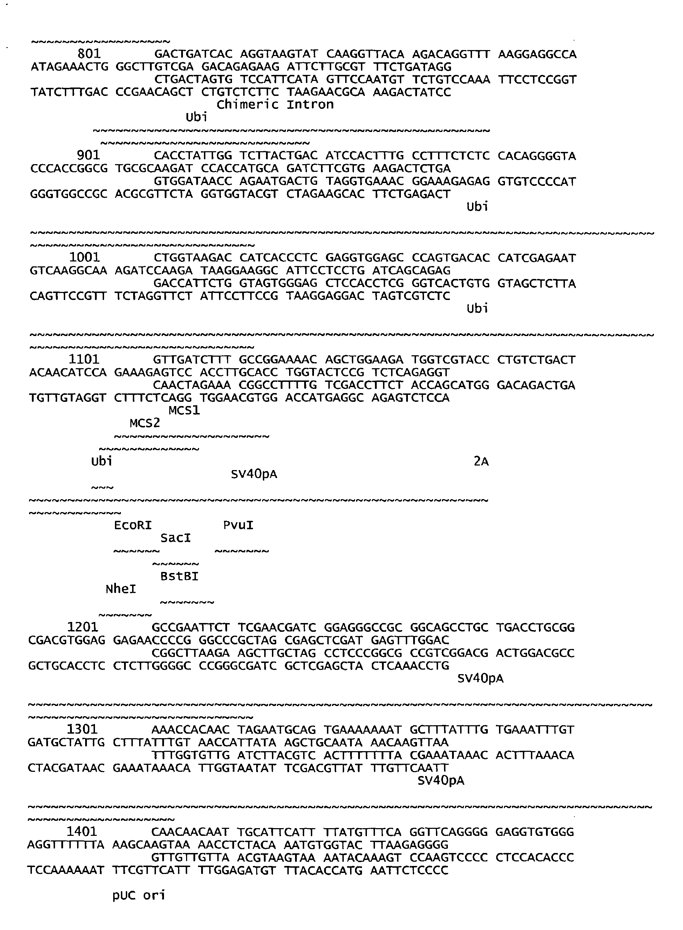

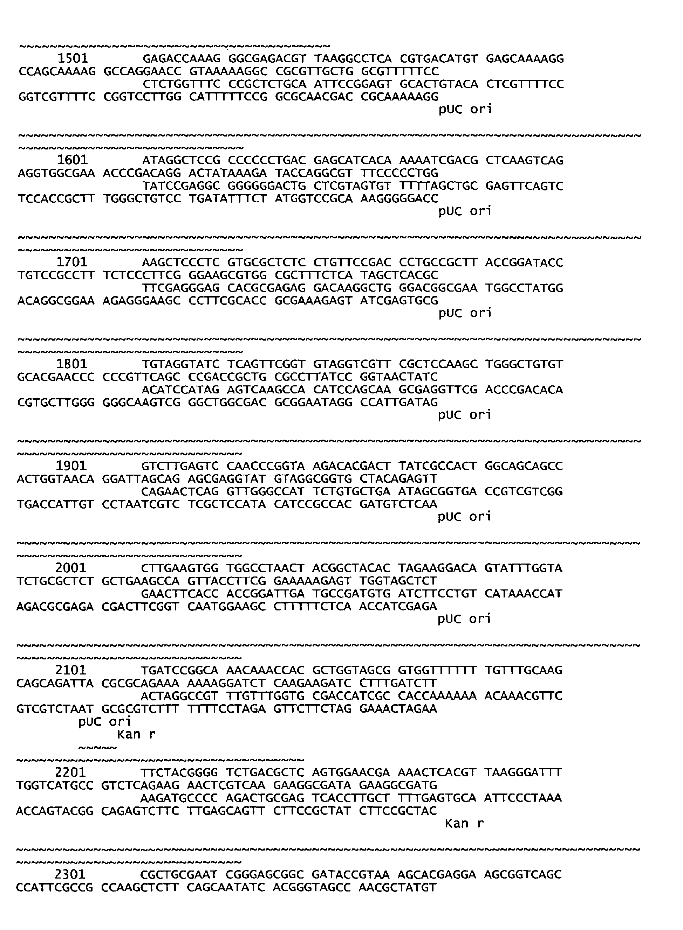

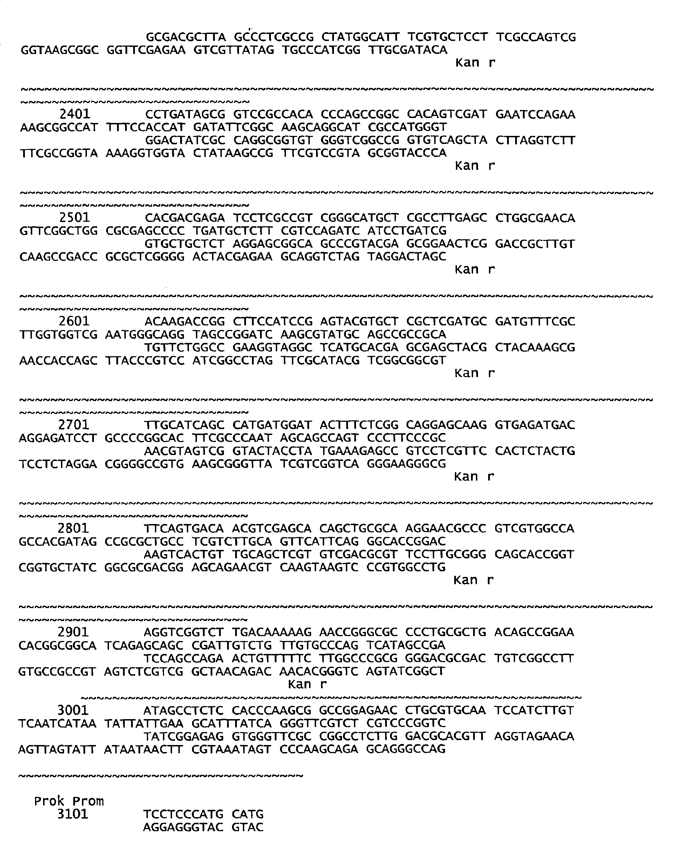

- Figure 18:: Module-based vector pC-Ubi-MCS1-2A-MCS2 (schematic) (Figure 18A) and corresponding nucleic acid sequence (SEQ ID NO: 11 and SEQ ID NO: 12, re- spectively) (Figure 18B).

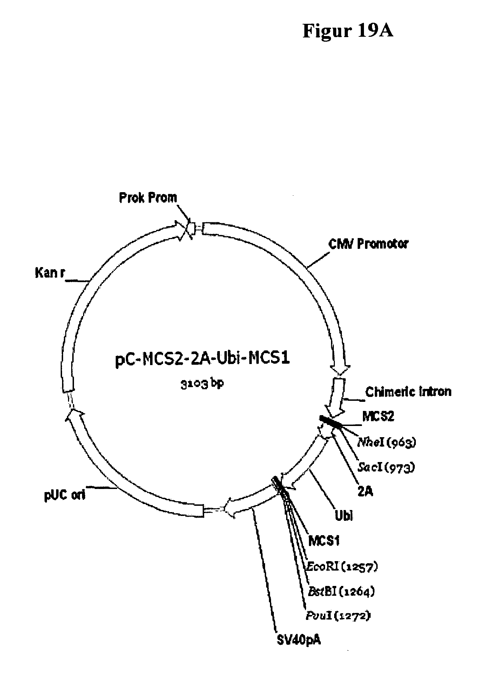

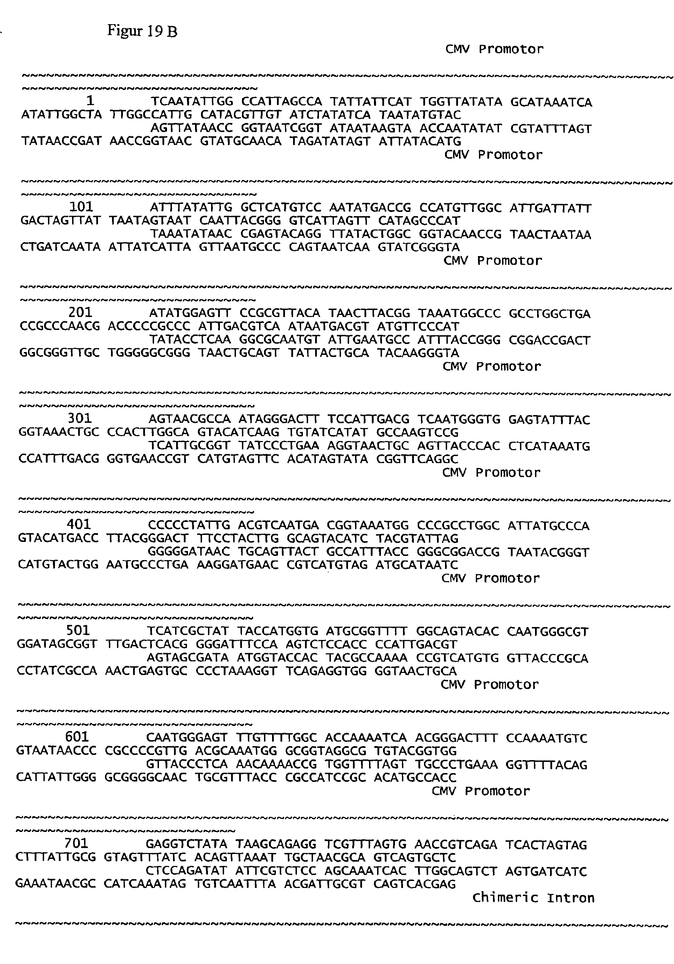

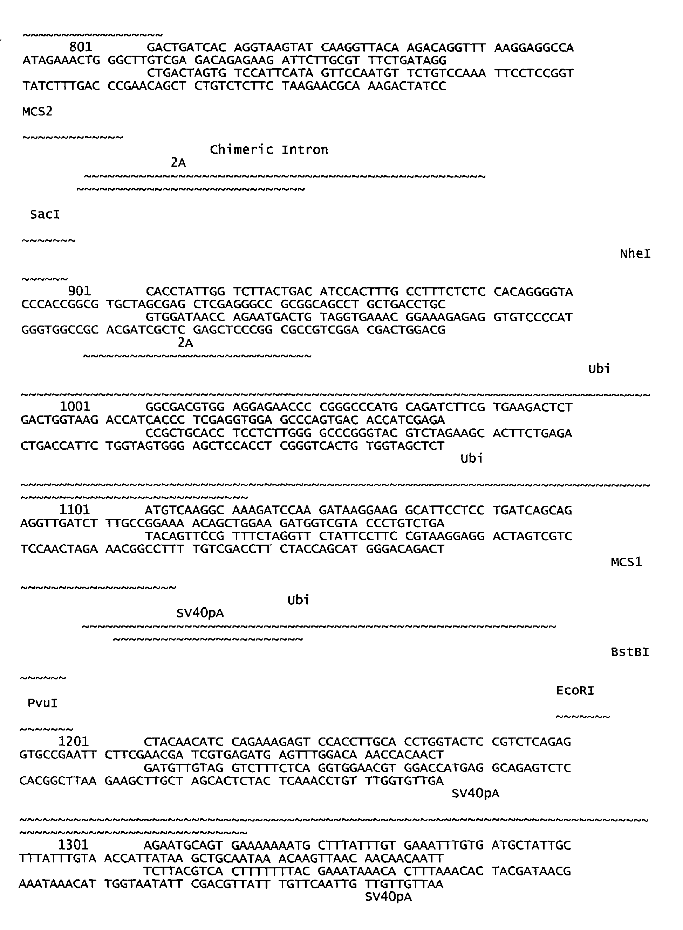

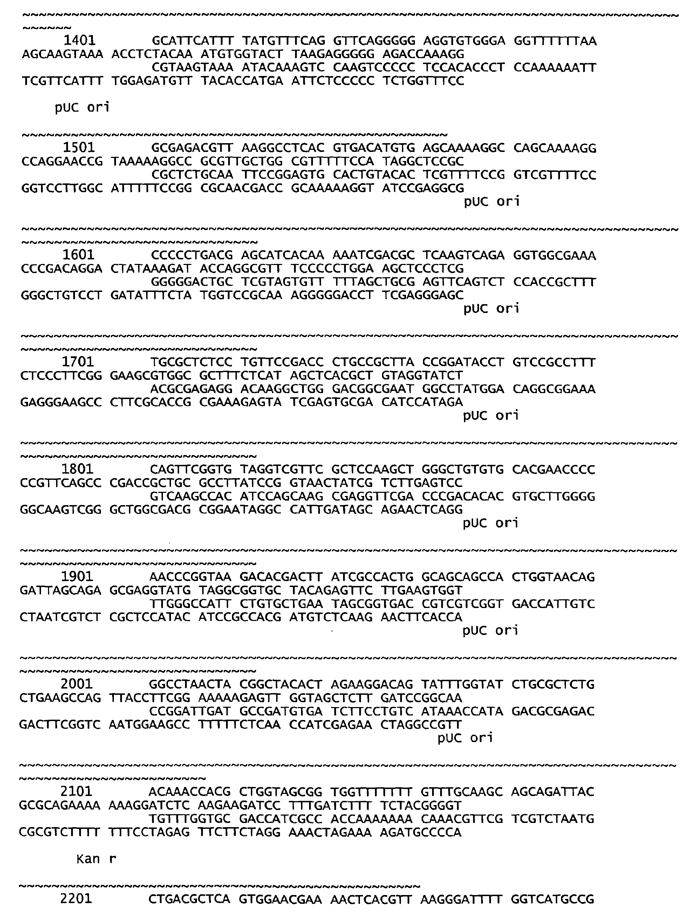

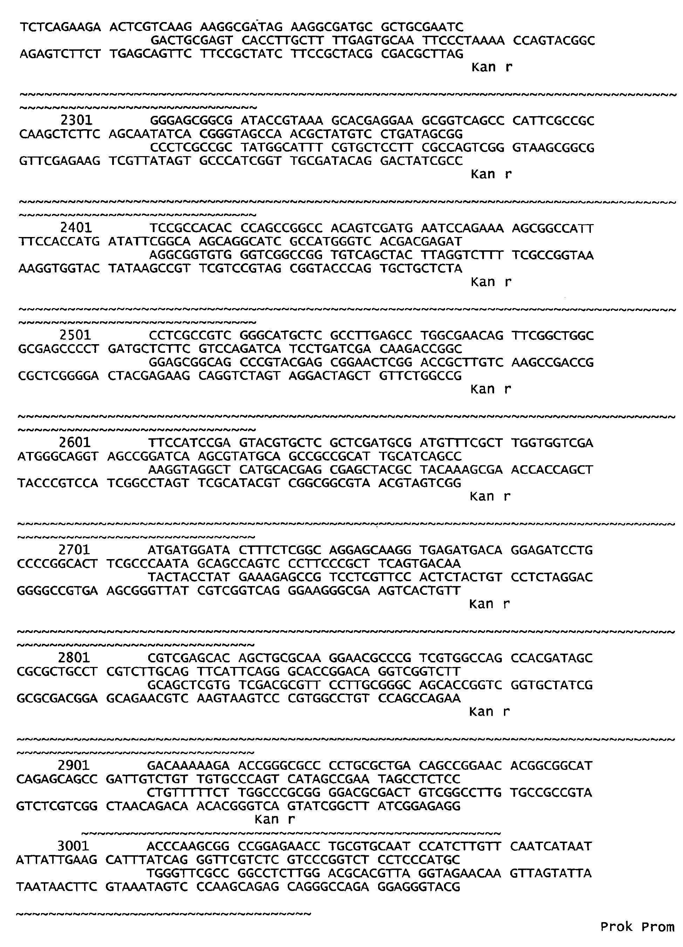

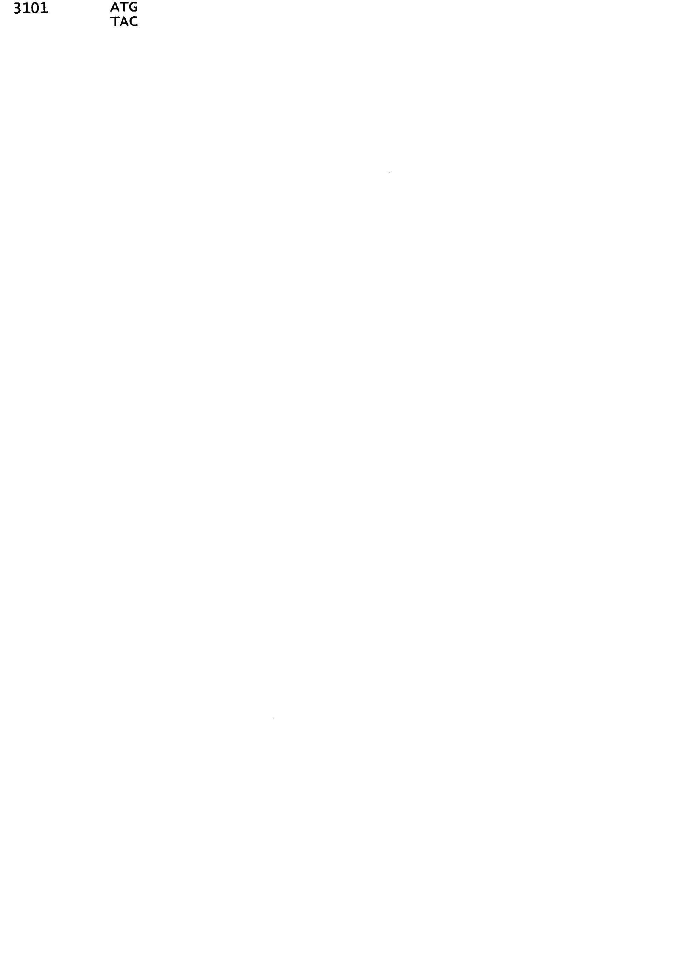

- Figure 19:: Module-based vector pC-MCS2-2A-Ubi-MCS1 (schematic) (Figure 19A) and corresponding nucleic acid sequence (SEQ ID NO: 13 and SEQ ID NO: 14, re- spectively) (Figure 19B).

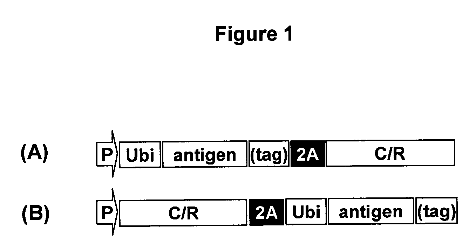

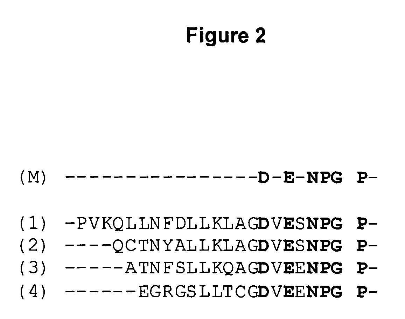

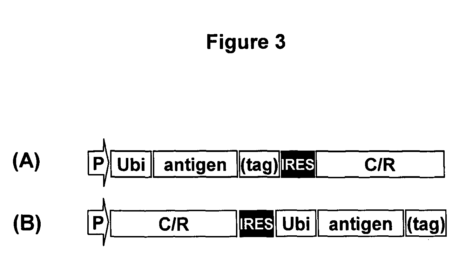

The module-based SV and RV for expression of a PDP with upstream Ubi as shown in Fig. 1 allows for immediate proteasomal degradation of directly fused downstream protein and a functionally uncoupled second protein that might be a prerequisite membrane-bound co-stimulatory molecule for antigen-specific T cell activation and/or a fluorescent reporter protein to assess T cell functions. Functional uncoupling ensures that only the PDP is degraded upon expression while the concomitant C/R protein once expressed, remains on the cell surface or within the cytoplasma of the transfected cell. Expression cassettes cloned to obtain final expression vectors comprise coding sequences for the respective proteins with N-terminal Ubi only for the protein to be degraded by the proetasome, structures to uncouple the expression of both proteins and an optional short tag for protein detection/purification. As for functional uncoupling a fusion protein that contains a self-cleavage peptide sequence can be generated (Figs. 1, 2), or a fused mRNA with an IRES for independent translation with a single upstream promoter gives rise to both proteins (Fig. 3). In all constructs an optional tag at the C-terminus of the PDP might allow assessing expression levels of the given antigen. The tag sequence might code for a standard tag peptide that should not exceed 30 amino acid residues e.g. the hemagglutinin (HA)-tag, the myc-tag or the 6xHis-tag.

As illustrated in Fig. 1 proteins to be expressed using the SV/RV vectors may be translated as a fusion protein containing an internal self-degrading peptide sequence for functional uncoupling of the two proteins. Expression of comparable amounts of both proteins is ensured, independent of the order of the coding sequences downstream of the single strong promoter for eukaryotic expression. Promoters for e.g. the following sources can be used: human light/heavy-chain ferritin (hFerL, hFerH), rat or mouse elongation factor 1 (rEF1, mEF1), cytomegalovirus (CMV), human T lymphotropic virus (HTLV), Ubi, respiratory syncytial virus (RSV), β actin, simian virus 40 (SV40). The sequence for a self-cleaving peptide can be cloned either downstream of the C/R protein (Fig. 1A) or downstream of the optional tag and/or the antigen sequence (Fig. 1B). Minimum prerequisite amino acid residues for self-degrading peptide might be derived from viral polyprotein processing mechanisms e.g. from Picornaviridae (Fig. 2). Preferentially, a 2A sequence self-cleaving between adjacent glycine and proline residues at the C-terminus of the complete sequence is used (27).

As illustrated in Fig. 3 the antigenic PDP and the C/R protein may be transcribed independently from one mRNA comprising an IRES sequence. Both proteins are expressed independently of the order of the coding sequences downstream of a single strong promoter for eukaryotic expression, as exemplified above for the approach including the self-degrading peptide. The promoter can be cloned either downstream of the C/R protein (Fig. 3A) or downstream of the optional tag and/or the antigen sequence (Fig. 3B).

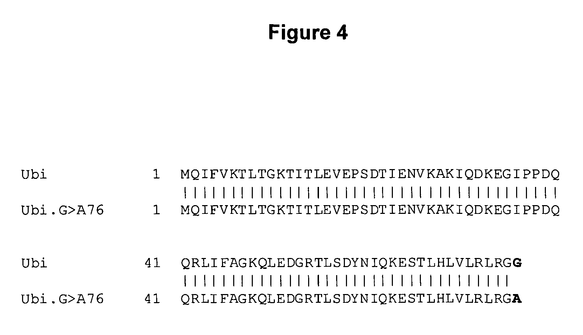

The Ubi sequence recombinantly fused to the N-terminus of the antigenic protein sequence sentences the protein to proteasomal degradation (28-30). It can be an unmodified Ubi sequence derived from any species or a mutated sequence with the C-terminal glycine residue replaced by a C-terminal alanine residue (Fig. 4) (31).

Any protein described to effectively assist interactions of APC with antigen-specfic T cells via the TCR can be used for a potential membrane-bound co-stimulatory molecule that is constitutively expressed to the surface of transfected cells. Typical examples of the type of such molecules are CD80, CD86, the ligand for the glucocorticoid-induced TNF receptor GITR-L, CD54, CD40, OX40 or 4-1BB, a ligand of a type 2 transmembrane glycoprotein of the TNF superfamily.

As for a reporter any fluorescent protein independent of excitation (λex) and emission wavelength (λem) can be used in the modular vector system to assess effector T cell functions. Preferentially, fluorescences at λem should be sufficiently discriminated by the readout method chosen, e.g. a concomitant use of a green fluorescent protein (GFP; λex = 475 nm λem = 505 nm) and of a red fluorescent protein (dsRed; λex = 557 nm λem = 579 nm) for flow cytometric analysis. Due to the mode of expression, fluorescence intensity of the reporter protein is directly proportional to the antigenic PDP expression.

Sequences, e.g. a viral T7 promoter allowing for in-vitro transcription to RNA might be included upstream of the cassettes shown in Figs. 1, 3 to use transcribed RNA instead of plasmid DNA to transfect cells that benefit from this strategy, e.g. primary cells including PBMC (32,33).

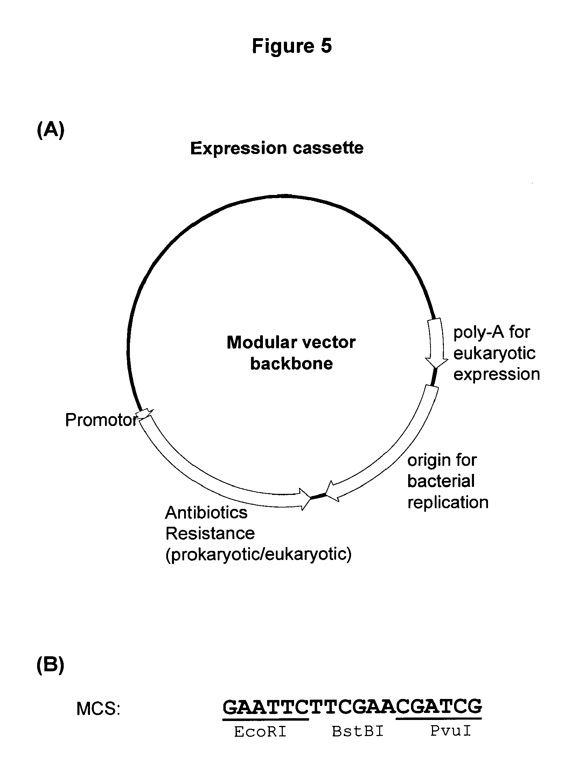

The backbone for final expression vectors typically comprises at least one antibiotics resistance gene for selection in bacteria and/or eukaryotic cells, an origin for bacterial replication, a poly-A sequence for eukaryotic expression downstream of the sequences for recombinant PDP and/or C/R protein (Fig. 5). Optionally, the backbone contains an origin for episomal eukaryotic replication or a sequence for directed chromosomal recombination flanking the cassettes shown in Fig. 1, 3. Antigenic PDP are cloned in a multiple cloning site (MCS) as exemplified in Fig. 5B.

The preferred method for transfection of potential APC with SV or potential target cells with RV as described above depends on the size of the final vector construct and the cell type to be transfected. Any established transfection method e.g. chemical precipitation, lipofection, electroporation or nucleofection can be used to transfect at least 20% of the cells to express the cloned and functionally uncoupled PDP and C/R protein.

The SV and RV as described above are used in PTF assays including previous antigen-specific in-vitro activation and/or expansion of effector T cells from samples with low initial frequencies or overall small amounts of these T cells. The procedure includes an antigen-specific stimulation of CTL from mixed cell populations using stimulating cells transfected for specific antigen expression as a PDP in conjunction with an uncoupled membrane co-stimulatory molecule followed by a PTF assay using target cells transfected for expression of the specific antigenic PDP used for preceding stimulation or a control PDP, in conjunction with uncoupled fluorescent reporter proteins that can be differentiated experimentally by diverse λem. In a multi parameter fluorescent readout transfected target cells as well as effector cells can be additionally characterized by cell-subtype specific surface marker antigens e.g. CD4, CD8 or CD137.

### EXAMPLES

### EXAMPLE 1: Different cell types can be effectively transfected with the vector constructs to be used as APC for T cell stimulation and as target cells in PTF assays.

Autologous B-LCL comprise the complete HLA pattern of the donor. Although with these cells strong EBV-specific T cell responses might overlap, the PTF assay allows to discriminate effector CTL for other antigens. The erythroleukemia cell line K562 (ACC 10; Deutsche Sammlung für Mikroorganismen und Zellkulturen, Braunschweig, Germany) does not express HLA class I molecules, but can intracellularly process proteins. Recombinant constitutive expression of e.g. HLA-A2 in K562 cells (17) provide a defined tool to assess HLA-A2 associated effects on CD8+ T-cell activation and subsequent identification of immunogenic peptides of a given antigen. PBMC are a heterogeneous primary cell population with the donor's HLA pattern. Although PBMC can easily be obtained from anti-coagulated peripheral blood samples by density gradient centrifugation (e.g. GE Healthcare, Munich, Germany), frequencies of professional APC are typically below 10% of all PBMC. The lymphoid EL-4 cell line (ATCC TIB 39; American Type Culture Collection, Bethesda, MD) can be used as a tool in autologous experimental settings in the murine C57BL/6 model system.

Using a Nucleofector I device (Lonza, Cologne, Germany) according to the manufacturer's protocol 2-5x10⁶ cells were transfected with 5 µg highly purified plasmid DNA for expression of fusion proteins of a given antigen with the GFP. After 24 h at 37°C, transfection efficiencies were determined by flow cytometry and are shown as percentages of GFP+ cells within all vital cells (Table 1).

**Table 1: Transfection efficiencies for potential stimulatory and target cells**

| Cell type | Nucleofection | | Antigen | GFP+ cells (%) | | N |
|---|---|---|---|---|---|---|
| | Kit | Program | | Mean | Range | |
| B-LCL | T | U01 | HPV16E7 | 40% | 20-60% | >10 |
| K562 | V | T16 | HPV16E7 | 90% | 85-95% | >30 |
| K562-A2 | V | T16 | HPV16E7 | 90% | 85-95% | >30 |
| PBMC | T cell Kit | U14 | HPV16E7 | 45% | 25-65% | >10 |
| EL-4 | V | C09 | HPV16E7 | 75% | 65-80% | >10 |

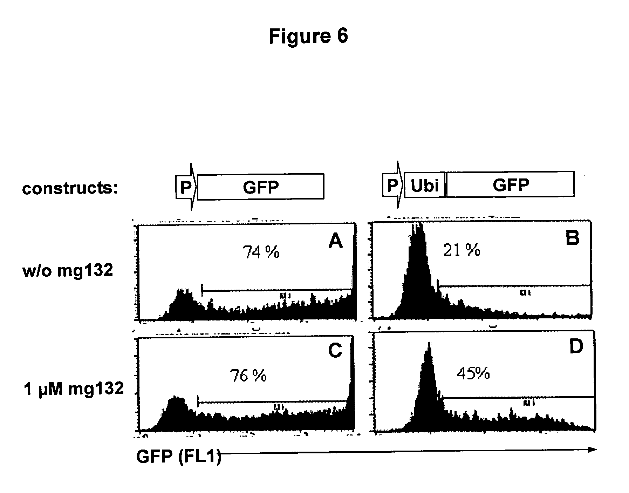

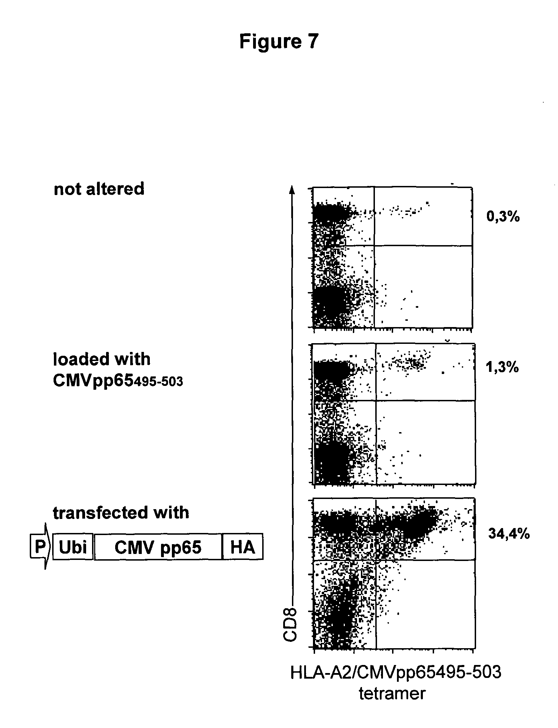

### EXAMPLE 2: N-terminal Ubi triggers proteasomal degradation of downstream fused proteins in transfected K562 cells and allows for specific antigen-presentation (Figs. 6 and 7).

Coding sequences for a GFP (Gene Bank accession number: AY268072) were cloned in frame downstream to Ubi or without the Ubi sequence. K562 cells (2x10⁶) from standard culture were transfected with 5 µg of the respective highly purified plasmid DNA as described for Example 1. Transfection efficiencies determined by flow cytometry after 24 h were about 75% GFP+ K562 cells as shown by cells transfected with the GFP construct (Fig. 6A). N-terminal Ubi reduced detectable GFP+ cells to 21% (Fig. 6B). Proteasome dependency of detectable GFP was assessed using the proteasome inhibitor mg-132 (Sigma-Aldrich, Hamburg, Germany) and 8 h after transfection with the GFP or the Ubi-GFP construct cultures received 1 µM mg-132 for additional 16 h. While mg-132 did not affect GFP in the cells transfected with GFP alone (Fig. 6C), percentages of GFP+ K652 cells were 45% for the Ubi-GFP construct (Fig. 6D) and at least two times higher than in the setup without the inhibitor.

A coding sequence for the phosphoprotein 65 from CMV (CMVpp65; Gene Bank accession number: M15120), known to elicit specific CD8+ T cell reactions in the context of HLA-A2, was cloned in frame downstream to the human Ubi sequence to form a SV. Autologous DC (1x10⁶) were transfected with 5 µg highly purified plasmid DNA by nucleofection as described above. Alternatively, 1x10⁶ DC were preincubated with 10 µg/ml of the CMVpp65-derived HLA-A2-specific peptide CMVpp65₄₉₅₋₅₀₃ (NLVPMVATV [SEQ ID NO: 2]; Biosyntan, Berlin, Germany) for 3 h at 37°C. Cultures with 1x10⁷ freshly prepared PBMC and 1x10⁶ SV-transfected or peptide-loaded DC cells were set up in 6-well tissue culture plates and incubated at 37°C for 7 days. For control, non-treated DC were used in co-cultures with PBMC. Cells were stained for CMVpp65₄₉₅₋₅₀₃-specific CD8+ T cells by flow cytometry after surface staining with a CD8-specific monoclonal antibody (clone RPA-T8; Becton Dickenson, Franklyn Lakes, USA) and HLA-A2/CMVpp65₄₉₅₋₅₀₃ tetramer (kind gift of S. Stevanovic, Tübingen, Germany) staining (Fig. 7). DC without specific antigen did not function as APC and induced a background of 0.1% CD8+ tetramer+ T cells (range: 0.03 -0.15%; n = 3). With respect to antigen-specific CD8+ T-cell activation, Ubi-CMVpp65-transfected APC (range: 15.3-34.4%; n = 3) were superior to APC loaded with the CMVpp65₄₉₅₋₅₀₃ peptide (range: 1.1-1.5%; n = 3).

Since the proteasome inhibitor partially restored the GFP enrichment in the Ubi-GFP expressing K562 and autologous DC transfected with an Ubi-antigen construct induced antigen-specific T cells, the recombinant fusion of N-terminal Ubi to a defined protein indeed produced a PDP that fulfilled the prerequisites for MHC/HLA-dependent presentation of immunogenic peptides.

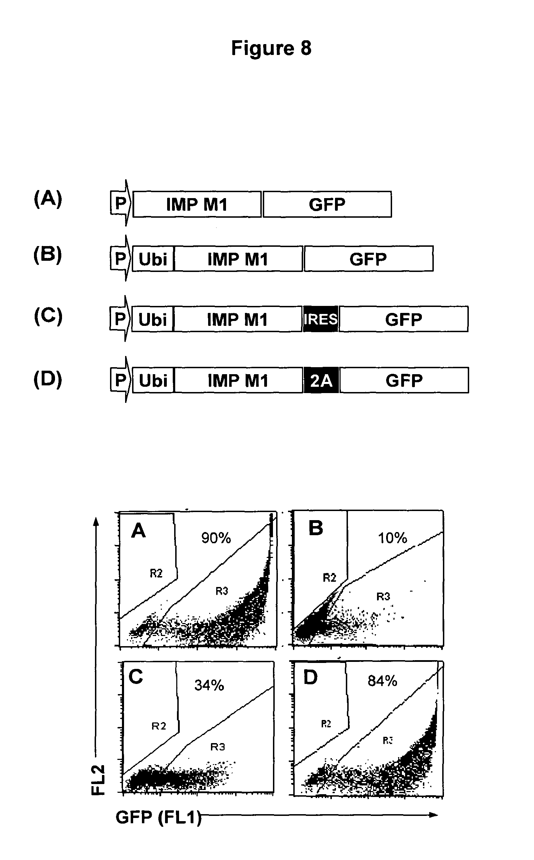

### EXAMPLE 3: Recombinant uncoupling of ubiquitinated influenza matrix protein IMP M1 and GFP prevents GFP degradation in transfected K562 cells (Fig. 8).

Coding sequences for IMP M1 (Gene Bank accession number: M63527) and GFP were cloned as a standard fusion protein (Fig. 8A, upper panel), as a proteasome-degradable fusion protein with upstream Ubi (Fig. 8B, upper panel), and for PDP and GFP uncoupled at translational level by the IRES sequence (Fig. 8C, upper panel) or for post-translational self-cleavage via the 2A site (Fig. 8D, upper panel). K562 cells (2x10⁶) were transfected with 5 µg of the respective highly purified plasmid DNA by nucleofection as described for Example 1 and cultured for 24 h. As assessed by flow cytometry, about 90% (range: 87-92%; n = 2) of the cells transfected with the standard fusion protein with C-terminal GFP were GFP+ (Fig. 8A, lower panel). Upstream human Ubi triggered PDP degradation as measured by dramatically reduced percentages of GFP+ cells (range: 8.5-9.6%, n = 2; Fig. 8B, lower panel).

While both strategies for uncoupling PDP and GFP worked nicely to recover GFP+ cells, the 2A was superior (range: 83-85%; n = 2; Fig. 8D, lower panel) compared to the IRES sequence (range: 32-35%; n = 2; Fig. 8C, lower panel).

Both, IRES and 2A sequences sufficiently uncouple the PDP from the fluorescent protein. Thus, a stabilized reporter allows for fluorescence-based readout systems to assess antigen-specific actions on transfected APC that depend on intracellular antigen processing.

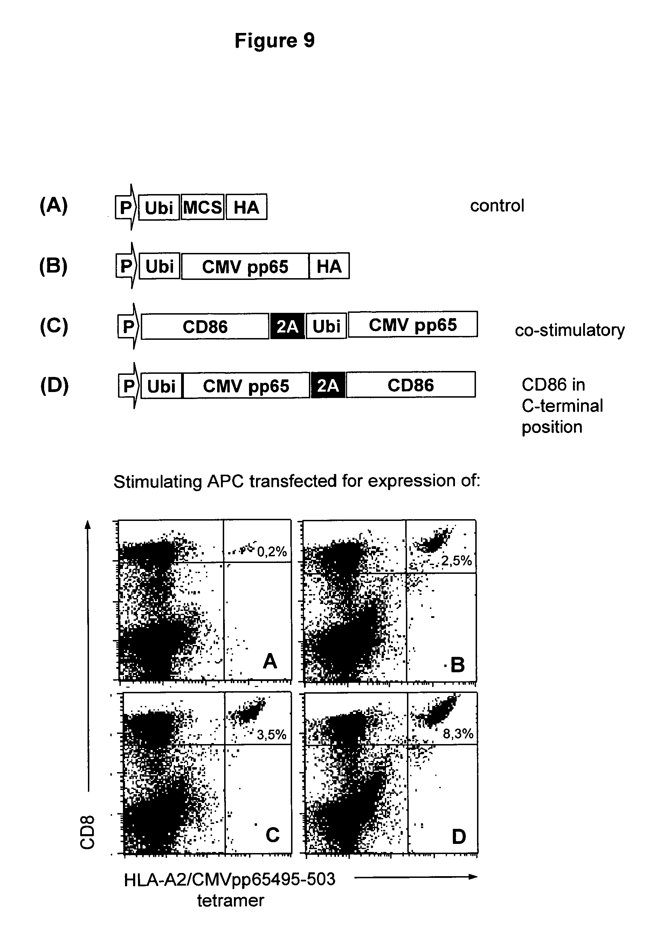

### EXAMPLE 4: Stimulating cells transfected for expression of ubiquitinated CMVpp65 in conjunction with uncoupled CD86 are superior to ubiquitinated CMVpp65 expression alone in CMVpp65-specific CD8+ T-cell activation (Fig. 9).

As shown in the upper panels of Fig. 9, a coding sequence for CMVpp65 was cloned in frame downstream to the Ubi sequence (Fig. 9B). For a control SV, the Ubi sequence was directly fused to the HA-tag (Fig. 9A). For a co-stimulatory SV, the CMVpp65 sequence was combined with a sequence for human CD86 (Gene Bank accession number: NM175862) uncoupled by use of a self-cleaving 2A site (Fig. 9C, D). Freshly isolated PBMC (5x10⁶) were transfected with 5 µg of the respective highly purified plasmid DNA by nucleofection as described for Example 1. Cultures of 1x10⁷ freshly prepared PBMC and 2x10⁶ SV-transfected PBMC were set up in 6-well tissue culture plates and incubated at 37°C for 7 days. Cells were stained for CMVpp65 specific CD8+ T cells by flow cytometry after staining with the CD8-specific monoclonal antibody (clone RPA-T8) and the HLA-A2/CMVpp65495-503 tetramer (Fig. 9, lower panels). PBMC transfected with the control vector did not function as APC and induced no background (Fig. 9A). With respect to antigen-specific CD8+ T-cell activation, PBMC transfected with antigen construct coupled to CD86 was more effective (Fig. 9C, D) than PBMC transfected with the antigen only (Fig. 9B). The co-stimulatory potential of CD86 in the C-terminal position of the SV-vector was superior to N-terminal CD86, with respect to antigen-specific T-cell activation.

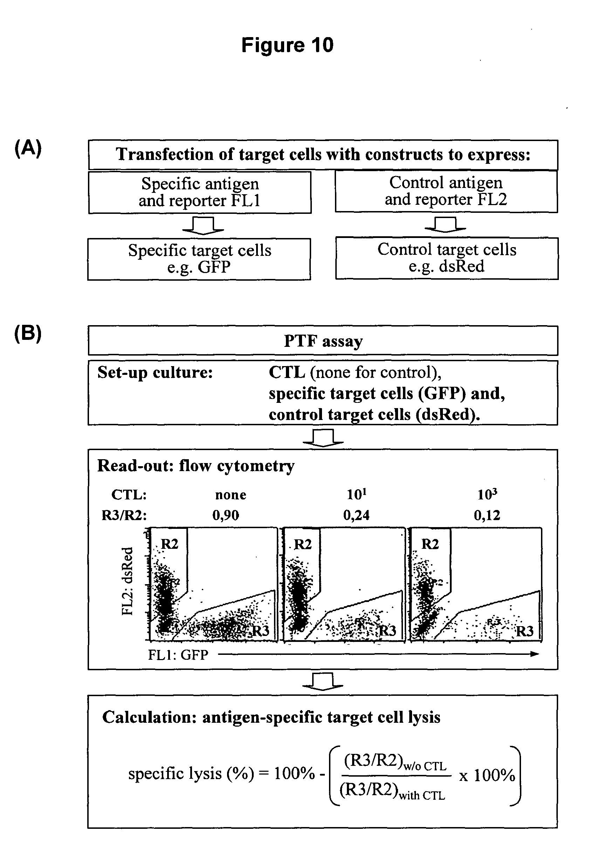

### EXAMPLE 5: Cells transfected for expression of ubiquitinated specific antigens in conjunction with uncoupled stable fluorescent reporter proteins and of ubiquitinated control antigens in conjunction with different uncoupled stable fluorescent reporter proteins can be used as target cells in antigen-specific PTF assays (Figs. 10-12).

As for standard PTF assay, two types of target cells are generated by transfection with an RV (Fig. 10A). Specific target cells receive a RV coding for the ubiquitinated antigenic protein and a fluorescent reporter e.g. GFP. Control target cells are transfected for expression of an irrelevant control protein as PDP and a different fluorescent reporter e.g. dsRed. These cells can be used as target cells for CTL either directly after transfection or after intermittent culture for 24 h at 37°C. Optimum readout requires about 50% (range: 20-99%) transfected target cells in both populations. Defined constant numbers of specific and control target cells are mixed with defined titrated numbers of CTL (Fig. 10B). Dependent on the cell type and/or initial frequencies of specific cells to be questioned for cytotoxic functionality, cultures are incubated for 24-96 h. Target cell mixtures without CTL control fluorescent reporter stability. As for CTL activity readout, fluorescent cells are assessed by flow cytometry. If present, antigen-specific CTL lyse transfected specific target cells resulting in reduced amounts of GFP+ cells in comparison to the control target cells. Specific lysis can be calculated from the ratios of GFP+ and dsRed+ cells from cultures with or without CTL.

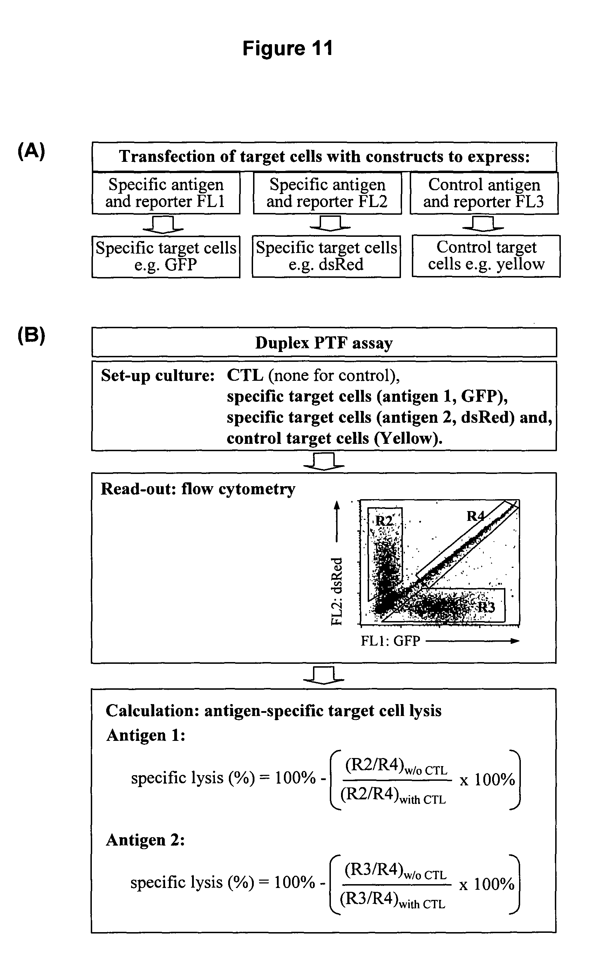

Set-ups for duplex PTF assays are similar to the standard PTF procedures described above. As for a main difference, two different specific target cell types are generated by transfection for expression of different specific antigens as PDP in conjunction with respective different fluorescent reporter proteins, e.g. specific antigen 1 with GFP and specific antigen 2 with dsRed. A third fluorescent reporter protein e.g. Yellow is coupled to the ubiquitinated irrelevant control protein (Fig. 11A). Duplex PTF assay allow to directly compare antigenicity of two independent proteins using the same CTL preparation (Fig. 11B). This approach is especially suitable if numbers of specific CTL available are low and to map immunogenic epitopes of given antigenic proteins.

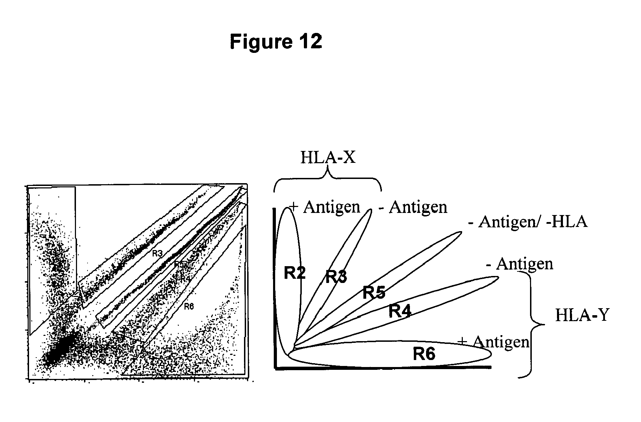

Recombinant fusion of fluorescent proteins e.g. to Yellow-Red or Yellow-Green provides additional reporters to be discriminated in flow cytometry and establishes the platform for multiplex PTF assays e.g. to investigate the HLA context of immuno-dominant epitopes derived from intracellularly processed and presented antigenic proteins (Fig. 12). Defined HLA patterns of APC e.g. might be obtained using K562 cells that are modified to express single HLA variant molecules and the antigen of interest using the respective set of SV and RV.

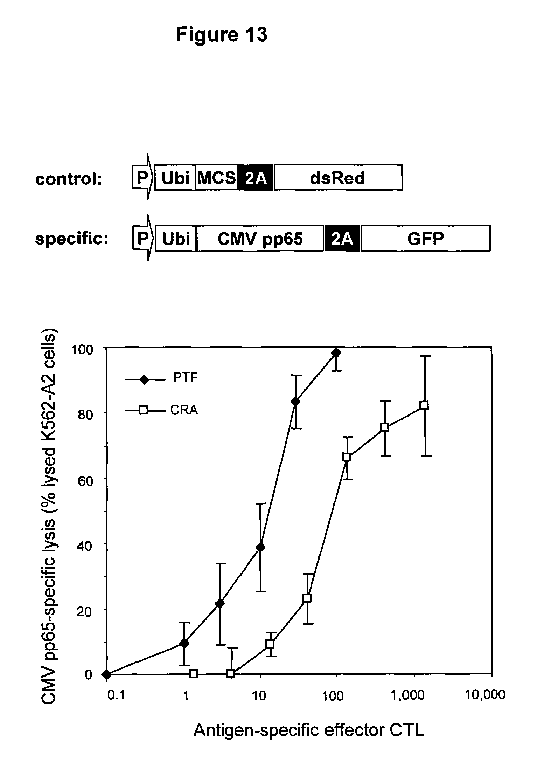

### EXAMPLE 6: Standard PTF assay using the K562-A2 CMVpp65 expressing target cells is about 10fold more sensitive in detection of CMVpp65-specific CTL compared to standard CRA using the specific peptide-loaded target cells (Fig. 13).

T cells specific for the CMVpp65₄₉₅₋₅₀₃ peptide were expanded from PBMC preparations within 7 days by in-vitro stimulation with autologous DC loaded with 10 µg/ml synthetic CMVpp65₄₉₅₋₅₀₃ peptide. The frequency of the antigen specific CMVpp65₄₉₅₋₅₀₃ as determined with HLA-A2/CMVpp65₄₉₅₋₅₀₃ tetramer staining typically were about 1.5% of all CD8+ T cells.

For generation of target cells the coding sequence of CMVpp65 as described in Example 2, was cloned into a RV comprising the Ubi, the self-cleaving 2A and a GFP reporter sequence. As for control RV, a vector including the dsRed sequence (dsRed Express 2, PT4078-5; Clontech, Mountain View, USA) was applied. K562-A2 cells (2x10⁶) were transfected with 5 µg of the respective highly purified plasmid DNA either with the control RV or the CMVpp65-expressing RV target by nucleofection as described for Example 1 and cultured for 24 h at 37°C. Up to 1x10⁴ CD8+ T cells specific for CMVpp65₄₉₅₋₅₀₃ per well as described above were titrated into 96-well V-bottom tissue culture plates. Transfected specific and control target cell populations were adjusted to comparable cell concentrations and mixed in equal amounts. Mixtures containing 1x10³ cells of each target cell type were distributed into the wells already containing the T cells. Wells containing target cells only were used as a control. For a PTF assay ratios of the specific and control target cell populations were directly measured by flow cytometry after incubation for 48 h. The number of CMVpp65₄₉₅₋₅₀₃-specific CD8+ T cells was correlated with the antigen specific target lyses cultures. To set up of the CRA, [⁵¹Cr]-labelled K562-A2 target cells were loaded with 10 µg/ml synthetic CMVpp65495-503 peptide, whereas control cells were loaded with 10 µg/ml HIV Pol₅₁₀₋₅₁₈ (ILKEPVHGV [SEQ ID NO: 3]; Biosyntan, Berlin, Germany) and co-incubated for 6 h with the antigen specific CD8+ T cells.

While the CRA needed about 50 antigen-specific T cells to detect a significant antigen-specific target cell lysis, about 5 antigen-specific T cells resulted in a similar target cell lysis using the PTF assay. Thus, in direct comparison, the PTF assay is at least 10fold more sensitive than the CRA 'gold standard' to detect antigen-specific CTL functionality.

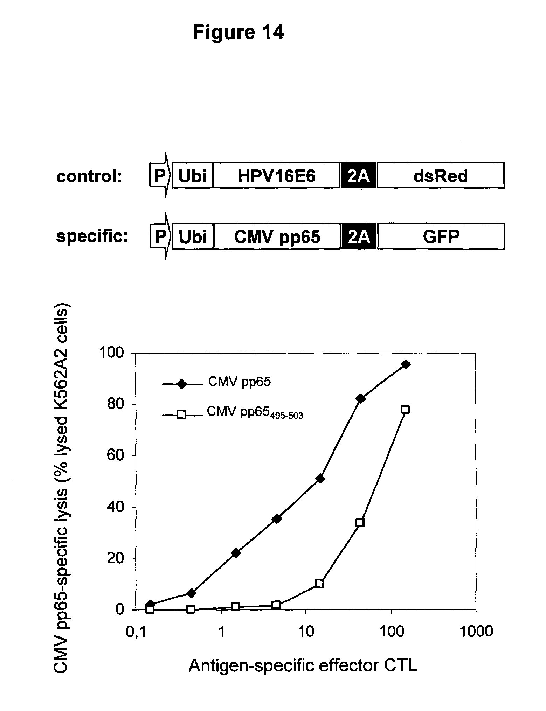

### EXAMPLE 7: Standard PTF assay applying CMVpp65-transfected target cells is superior in detection of CMVpp65-specific CTL compared to VITAL assay using CMVpp65₄₉₅₋₅₀₃-loaded target cells (Fig. 14).

Vector constructs and PTF assay set-up were applied as described in Example 6. To perform a VITAL assay a K562 A2 control and target cells population were stained with the fluorescent dyes carboxyfluorescein succinimidyl ester (CFSE) or Far Red as described by the manufacturer (Clontech, Mountain View, MA). The CFSE-stained target cells were loaded with 10 µg/ml CMVpp65₄₉₅₋₅₀₃ peptide while Far Red-stained control cells were loaded with 10 µg/ml HIV Pol₅₁₀₋₅₁₈. For co-incubation, CMVpp65-specific CTL were titrated into 96-well V-bottom plates ranging up to 1x10⁴ cells/well and were exposed to mixtures of 1x10³ control and target cells. After 48 h, specific lysis was determined from the ratio of control and target cells as directly measured by flow cytometry. Calculation of specific lysis was performed as for the PTF assay described for Fig. 10B. Directly comparing VITAL and PFA assays, antigen-specific target cell lysis with CMVpp65-transfected K562-A2 target cells was higher than with target cells loaded with synthetic peptides. Consequently only three CTL were necessary to detect an antigen-specific lysis in the PTF assay whereas 10fold more CTL were needed in the VITAL assay.

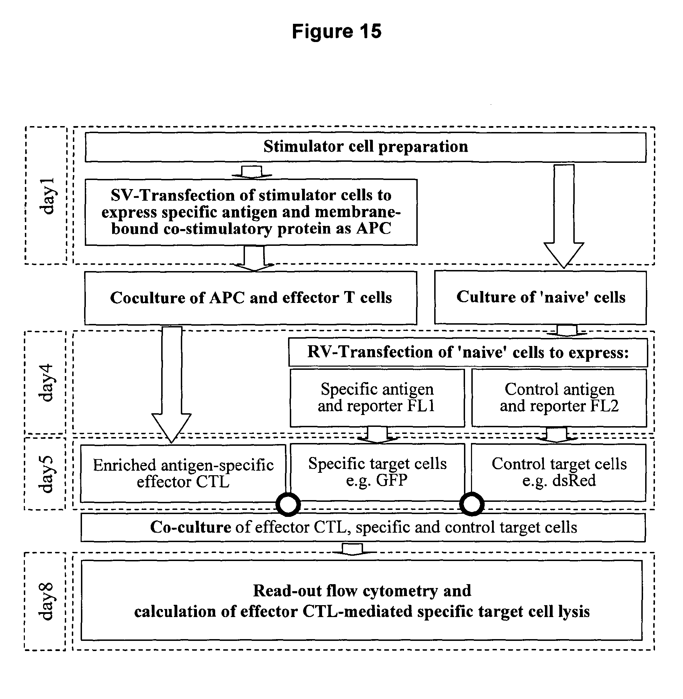

### EXAMPLE 8: Detection of functional CTL naturally occurring at low frequencies should include short-term antigen-specific T cell activation before subsequent PFA assay (Fig. 15).

A standard assay for the fast detection of functional CTL occurring at low frequencies might combine the methods described in Examples 4 and 5. A primary cell population e.g. freshly isolated PBMC or TIL are divided. One part is transfected with a SV harbouring the antigen of interest in combination with a co-stimulatory molecule and used as APC for autologous PBMC or TIL. The other part is cultured without further treatment. At day 4, these untreated cells are transfected separately with either a RV containing the same specific antigen as the SV above or with a respective control RV. The following day, cell population stimulated with SV-transfected APC and therefore enriched in antigen-specific T cells is mixed with the RV-transfected specific and the control target cell population. After a co-culturing period of 24-72 h the specific lysis of the antigen-specific target cell population is determined by flow cytometry as described above.

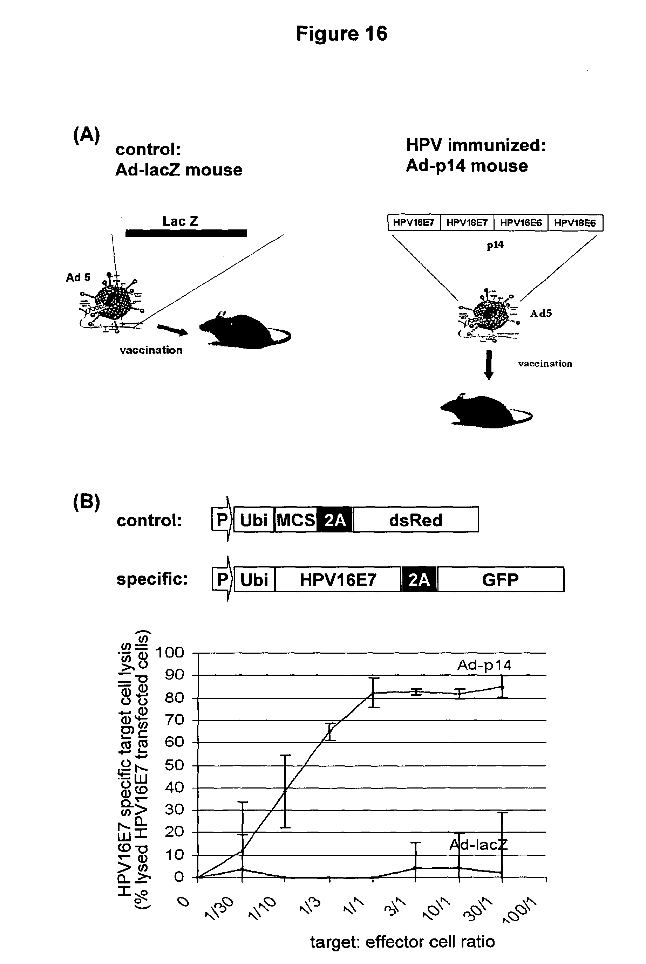

### EXAMPLE 9: Immunization of C57/BL6 mice with a therapeutic HPV vaccine induces low frequencies of HPV16E7-specific CTL analysed by standard PTF assay (Fig. 16).

For immune monitoring studies, two groups of mice were immunized with different vaccines (Fig. 16A). The regional animal study committee of Berlin, Germany approved animal protocols. One group received 1x10¹⁰ infectious particles of the therapeutic HPV vaccine Ad-p14, targeting HPV16/18, the other group 1x10¹⁰ infectious particles of the control vaccine Ad-LacZ. Twelve days after immunisation PBMC were isolated from mice spleens. For in-vitro activation vaccine-induced 5x10⁶ CTL were co-incubated with irradiated 5x10⁵ EL-4 cells expressing the HPV16E7 antigen. After in-vitro restimulation for 7 days, CD8+ T cells were tested in the PTF assay for cytotoxic activity.

A coding sequence for antigen HPV16E7 (Gene bank accession number: AF536180) was cloned in RV comprising the reporter sequence GFP (Fig. 16B). The control RV included the dsRed reporter sequence. For the PTF assay, 1x10⁶ EL-4-cells were transfected with 5 µg of the respective highly purified plasmid DNA of control or specific RV by nucleofection as described in Example 1. The experimental set-up and analysis corresponded to the performance specified in Example 6. About 100 deployed CD8+ T cells of Ad-p14-immunized mice resulted in a significant lysis of HPV16E7-expressing target cells. Equal numbers of CD8+ T cells obtained from control mice did not result in a specific lysis.

Thus, the highly sensitive PTF assay detected reproducibly detected vaccine-induced HPV-specific CD8+ T cells. Consequently, the PTF assay allows a direct immune monitoring in vaccination studies.

### EXAMPLE 10: HPV16E6-specific stimulation of CTL from autologous TIL preparations allows detection of low frequencies of HPV 16E6-specific CTL by standard PTF assay (Fig. 14).

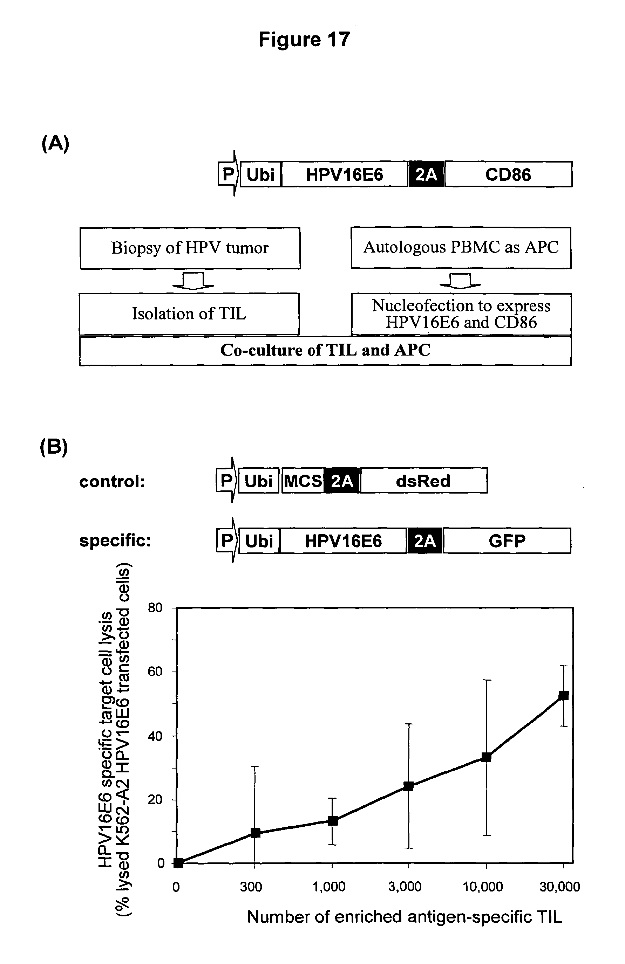

With informed consent and with the approval of the local ethics committee of the Charité - Universitätsmedizin, Berlin, Germany, TIL were prepared from biopsy of a HPV16+ tumor from HLA-A2+ patient and tested for HPV16E6-specific CTL. The coding sequence of HPV16E6 (Gene bank accession number: AF536180) was cloned to obtain a SV comprising the ubiquitinated antigen in conjunction with CD86 functionally uncoupled via the 2A self-cleavage sequence (Fig. 17A). TIL (5x10⁶) were co-cultured with 5x10⁶ SV-transfected autologous PBMC as APS. Additionally, the HPV16E6 sequence was cloned into the RV with a functionally uncoupled GFP reporter (Fig. 17B). Six days later, 2x10⁶ K562-A2 cells were transfected with 5 µg of highly purified plasmid DNA of the HPV16E6-specific RV or a control RV for dsRed expression. At day 7, specific and control target cells were co-incubated with the restimulated TIL in a PTF assay. The HPV16E6-specific K562-A2 target cell lysis was correlated to the total number of TIL added to the assay. About 3x10³ cells from the expanded TIL population revealed specific lysis. Thus, the PTF assay according to the preferred embodiment of the invention is suitable to detect low frequencies of tumor-specific CTL from small samples within one week and can therefore be used to diagnostically assess tumor-specific immune responses.

### References

1 Dalgleish, A. and Pandha, H. 2007. Tumor antigens as surrogate markers and targets for therapy and vaccines. Adv Cancer Res 96:175-90.
2 Rosenberg, S. A., Spiess, P., and Lafreniere, R. 1986. A new approach to the adoptive immunotherapy of cancer with tumor-infiltrating lymphocytes. Science 233:1318-21.
3 Nguyen, D. D., Beck, L., and Spiegelberg, H. L. 1995. Anti-CD3-induced anergy in cloned human Th0, Th1, and Th2 cells. Cell Immunol 165:153-7.
4 Zinkernagel, R. M. and Doherty, P. C. 1974. Restriction of in vitro T cell-mediated cytotoxicity in lymphocytic choriomeningitis within a syngeneic or semiallogeneic system. Nature 248:701-2.
5 Fields, P., Fitch, F. W., and Gajewski, T. F. 1996. Control of T lymphocyte signal transduction through clonal anergy. J Mol Med 74:673-83.
6 Vasu, C., Wang, A., Gorla, S. R., Kaithamana, S., Prabhakar, B. S., and Holterman, M. J. 2003. CD80 and CD86 C domains play an important role in receptor binding and co-stimulatory properties. Int Immunol 15:167-75.
7 Purner, M. B., Berens, R. L., Krug, E. C., and Curiel, T. J. 1994. Epstein-Barr virus-transformed B cells, a potentially convenient source of autologous antigen-presenting cells for the propagation of certain human cytotoxic T lymphocytes. Clin Diagn Lab Immunol 1:696-700.
8 Hirano, N., Butler, M. O., Xia, Z., Berezovskaya, A., Murray, A. P., Ansen, S., and Nadler, L. M. 2006. Efficient presentation of naturally processed HLA class I peptides by artificial antigen-presenting cells for the generation of effective antitumor responses. Clin Cancer Res 12:2967-75.
9 Sasawatari, S., Tadaki, T., Isogai, M., Takahara, M., Nieda, M., and Kakimi, K. 2006. Efficient priming and expansion of antigen-specific CD8+ T cells by a novel cell-based artificial APC. Immunol Cell Biol 84:512-21.
10 Bellone, M., Iezzi, G., Manfredi, A. A., Protti, M. P., Dellabona, P., Casorati, G., and Rugarli, C. 1994. In vitro priming of cytotoxic T lymphocytes against poorly immunogenic epitopes by engineered antigen-presenting cells. Eur J Immunol 24:2691-8.
11 Yang, G., Hellstrom, K. E., Hellstrom, I., and Chen, L. 1995. Antitumor immunity elicited by tumor cells transfected with B7-2, a second ligand for CD28/CTLA-4 costimulatory molecules. J Immunol 154:2794-800.
12 Kaufmann, A. M., Gissmann, L., Schreckenberger, C., and Qiao, L. 1997. Cervical carcinoma cells transfected with the CD80 gene elicit a primary cytotoxic T lymphocyte response specific for HPV 16 E7 antigens. Cancer Gene Ther 4:377-82.
13 Larsen, M. V., Lundegaard, C., Lamberth, K., Buus, S., Brunak, S., Lund, O., and Nielsen, M. 2005. An integrative approach to CTL epitope prediction: a combined algorithm integrating MHC class I binding, TAP transport efficiency, and proteasomal cleavage predictions. Eur J Immunol 35:2295-303.
14 McMahan, R. H., McWilliams, J. A., Jordan, K. R., Dow, S. W., Wilson, D. B., and Slansky, J. E. 2006. Relating TCR-peptide-MHC affinity to immunogenicity for the design of tumor vaccines. J Clin Invest 116:2543-51.
15 Burgdorf, S. and Kurts, C. 2008. Endocytosis mechanisms and the cell biology of antigen presentation. Curr Opin Immunol 20:89-95.
16 Malyguine, A., Strobl, S., Zaritskaya, L., Baseler, M., and Shafer-Weaver, K. 2007. New approaches for monitoring CTL activity in clinical trials. Adv Exp Med Biol 601:273-84.
17 Britten, C. M., Meyer, R. G., Kreer, T., Drexler, I., Wolfel, T., and Herr, W. 2002. The use of HLA-A*0201-transfected K562 as standard antigen-presenting cells for CD8(+) T lymphocytes in IFN-gamma ELISPOT assays. J Immunol Methods 259:95-110.
18 Schoenborn, J. R. and Wilson, C. B. 2007. Regulation of interferon-gamma during innate and adaptive immune responses. Adv Immunol 96:41-101.
19 Xu, X. N. and Screaton, G. R. 2002. MHC/peptide tetramer-based studies of T cell function. J Immunol Methods 268:21-8.
20 Brunner, K. T., Mauel, J., Cerottini, J. C., and Chapuis, B. 1968. Quantitative assay of the lytic action of immune lymphoid cells on 51-Cr-labelled allogeneic target cells in vitro; inhibition by isoantibody and by drugs. Immunology 14:181-96.
21 Korzeniewski, C. and Callewaert, D. M. 1983. An enzyme-release assay for natural cytotoxicity. J Immunol Methods 64:313-20.
22 Devevre, E., Romero, P., and Mahnke, Y. D. 2006. LiveCount Assay: concomitant measurement of cytolytic activity and phenotypic characterisation of CD8(+) T-cells by flow cytometry. J Immunol Methods 311:31-46.
23 Hermans, I. F., Silk, J. D., Yang, J., Palmowski, M. J., Gileadi, U., McCarthy, C., Salio, M., Ronchese, F., and Cerundolo, V. 2004. The VITAL assay: a versatile fluorometric technique for assessing CTL- and NKT-mediated cytotoxicity against multiple targets in vitro and in vivo. J Immunol Methods 285:25-40.
24 Kienzle, N., Olver, S., Buttigieg, K., and Kelso, A. 2002. The fluorolysis assay, a highly sensitive method for measuring the cytolytic activity of T cells at very low numbers. J Immunol Methods 267:99-108.
25 Zweerink, H. J., Gammon, M. C., Utz, U., Sauma, S. Y., Harrer, T., Hawkins, J. C., Johnson, R. P., Sirotina, A., Hermes, J. D., Walker, B. D., and et al. 1993. Presentation of endogenous peptides to MHC class I-restricted cytotoxic T lymphocytes in transport deletion mutant T2 cells. J Immunol 150:1763-71.
26 van Baalen, C. A., Kwa, D., Verschuren, E. J., Reedijk, M. L., Boon, A. C., de Mutsert, G., Rimmelzwaan, G. F., Osterhaus, A. D., and Gruters, R. A. 2005. Fluorescent antigen-transfected target cell cytotoxic T lymphocyte assay for ex vivo detection of antigen-specific cell-mediated cytotoxicity. J Infect Dis 192:1183-90.
27 Donnelly, M. L., Hughes, L. E., Luke, G., Mendoza, H., ten Dam, E., Gani, D., and Ryan, M. D. 2001. The 'cleavage' activities of foot-and-mouth disease virus 2A site-directed mutants and naturally occurring '2A-like' sequences. J Gen Virol 82:1027-41.
28 Gaczynska, M., Rock, K. L., and Goldberg, A. L. 1993. Role of proteasomes in antigen presentation. Enzyme Protein 47:354-69.
29 Kloetzel, P. M. 2001. Antigen processing by the proteasome. Nat Rev Mol Cell Biol 2:179-87.
30 Zhang, M., Obata, C., Hisaeda, H., Ishii, K., Murata, S., Chiba, T., Tanaka, K., Li, Y., Furue, M., Chou, B., Imai, T., Duan, X., and Himeno, K. 2005. A novel DNA vaccine based on ubiquitin-proteasome pathway targeting 'self-antigens expressed in melanoma/melanocyte. Gene Ther 12:1049-57.
31 Qian, S. B., Ott, D. E., Schubert, U., Bennink, J. R., and Yewdell, J. W. 2002. Fusion proteins with COOH-terminal ubiquitin are stable and maintain dual functionality in vivo. J Biol Chem 277:38818-26.
32 Landi, A., Babiuk, L. A., and van Drunen Littel-van den Hurk, S. 2007. High transfection efficiency, gene expression, and viability of monocyte-derived human dendritic cells after nonviral gene transfer. J Leukoc Biol 82:849-60.
33 Yamamoto, A., Kormann, M., Rosenecker, J., and Rudolph, C. 2009. Current prospects for mRNA gene delivery. Eur J Pharm Biopharm 71:484-9.

### Abbreviations

| | |
|---|---|
| APC | antigen-presenting cell |
| B-LCL | EBV-transformed B lymphoblastic cell lines |
| CFSE | carboxyfluorescein succinimidyl ester |
| CMV | Cytomegalovirus |
| CRA | chromium release assay |
| CTL | cytotoxic CD8+ T cell |
| DC | dendritic cell |
| EBV | Epstein-Barr virus |
| ELISpot | enzyme-linked immunosorbent spot |
| FL1, FL2 | fluorescence channel |
| GFP | green fluorescent protein |
| HA | Hemagglutinine |
| HBV | Hepatitis B virus |
| HCV | Hepatitis C virus |
| HIV | human immunodeficiency virus |
| hFerL, hFerH | human light/heavy-chain ferritin |
| HTLV | human T lymphotropic virus |
| HLA | human leukocyte antigen |
| HPV | human papilloma virus |
| IC-FACS | flow cytometric assessment of intracellular cytokines |
| IFN | Interferon |
| IMP M1 | influenza matrix protein 1 |
| IRES | internal ribosomal entry site |
| λem | emission wavelength |
| λex | excitation wavelength |
| LDH | lactate dehydrogenase |
| MHC | major histocompatibility complex |
| mEF1 | Mouse elongation factor 1 |
| NK | natural killer |
| ORF | open reading frame |
| PDP | proteasome-degradable protein |
| PTF | plasmid transfection fluorolysis |
| rEF1 | rat elongation factor 1 |
| R2, R3 | gate |
| R/C | fluorescent reporter or a membrane-bound co-stimulatory protein |
| RSV | respiratory syncytial virus |
| RV | reporter vector |
| SV | stimulation vector |
| SV40 | simian virus 40. |
| TCR | T-cell receptor |
| TIL | tumor-infiltrating lymphocytes |
| TNF | tumor necrosis factor |
| Ubi | ubiquitin |

## Claims

1. A module-based vector for concomitant expression of an antigenic first protein and an accessory second protein, the vector comprising:
(a) a first nucleic acid sequence encoding a proteasome-degradable protein comprising a section encoding ubiquitin upstream of a section encoding the antigenic first protein;
(b) a second nucleic acid sequence encoding the accessory second protein;
(c) a third nucleic acid sequence for encoupling the antigenic first and the accessory second protein;
wherein the third nucleic acid sequence is located between the first and the second nucleic acid sequence; and
(d) a first promoter for eukaryotic expression which is located upstream of the first, second and third nucleic acid sequence.

2. The vector according to claim 1, wherein the first nucleic acid sequence is located upstream of the third nucleic acid sequence and the second nucleic acid sequence is located downstream of the third nucleic acid sequence, or wherein the first nucleic acid sequence is located downstream of the third nucleic acid sequence and the second nucleic acid sequence is located upstream of the third nucleic acid sequence.

3. The vector according to claim 1 or 2, wherein the third nucleic acid sequence is encoding an amino acid sequence comprising a self-cleavage peptide sequence, or wherein the third nucleic acid is encoding an mRNA comprising an internal ribosomal entry site.

4. The vector according to claim 3, wherein the third nucleic acid sequence is encoding an amino acid sequence comprising a self-cleavage peptide sequence having an amino acid sequence according to SEQ ID NO: 1, preferably a Picornavirus 2A self-cleavage sequence.

5. The vector according to any of the preceding claims, wherein the antigenic first protein is selected from the group comprising a tumor associated, viral, bacterial and autoantigenic protein.

6. The vector according to any of claims 1 to 5, wherein the accessory second protein is a reporter protein, preferably a fluorescent reporter protein, most preferably selected from the group comprising GFP, dsRed, Yellow and a recombinant combination thereof.

7. The vector according to any of claims 1 to 5, wherein the accessory second protein is a membrane-bound co-stimulatory protein for antigen-specific T cell activation, preferably selected from the group comprising CD80, CD86, GITR-L, CD54, CD40, OX40 and 4-1BB.

8. A target cell for CTL comprising a module-based vector according to claim 6.

9. A pair of target cells according to claim 8 consisting of a first target cell which is a test target cell, and a second target cell which is a control target cell, wherein the control target cell differs from the test target cell in the antigenic first protein and in the reporter protein.

10. An antigen-presenting cell comprising a module-based vector according to claim 7.

11. A method for preparing a target cell according to claim 8 or 9 comprising the step of transfecting a cell with a module-based vector according to claim 6.

12. A method for preparing an antigen-presenting cell according to claim 10 comprising the step of transfecting a cell with a module-based vector according to claim 7.

13. Use of a module-based vector according to claim 6, or of a target cell according to claim 8 or 9 for studying T cell functions *ex vivo,* preferably for detecting and monitoring T cells activated by a vaccine, an infectious agent, preferably a virus, an allergen or an autoantigen.

14. Use of a module-based vector according to claim 7, or of an antigen-presenting cell according to claim 10 for activating and/or expanding T cells.

15. A plasmid transfection fluorolysis assay comprising the following steps:
(a) providing one, two or more population(s) of a test target cell according to claim 9, preferably by using a method according to claim 11;
(b) providing a population of a control target cell according to claim 9, preferably by using a method according to claim 11;
(c) providing a population of CTL, preferably pre-activated by an antigenic protein expressed by the one, two or more population(s) of an antigen-presenting cell according to claim 10;
(d) mixing the populations of (a), (b) and (c);
(e) co-culturing the populations of (a), (b) and (c), preferably for 72 h;
(f) measuring by fluorescence spectroscopy, preferably by FACS, the extent of fluorescence for the one, two or more population(s) of a test target cell and the extent of fluorescence for the population of a control target cell;
(g) determining the ratio of the extent of fluorescence measured for each of the one, two or more population(s) of a test target cell and the extent of fluorescence measured for the population of a control target cell.
